# EUROPEAN PATENT APPLICATION

(11) **EP 2 589 383 A1**
(43) Date of publication of application: **08.05.2013**
(21) Application number: 11075245.8
(22) Date of filing: 06.11.2011
(51) Int. Cl.: A61K 31/436, A61P 35/00, A61P 7/02, A61P 25/00, A61P 25/28, A61P 3/04, A61P 25/24, A61P 5/50, A61P 3/10, A61P 37/06

(54) **FKBP subtype-specific rapamycin analogue for use in treatment of diseases**

(71) Applicant: Max-Planck-Gesellschaft zur Förderung der Wissenschaften e.V., 80539 München (DE)
(72) Inventor: Hausch, Felix, 80804 Munich (DE); März, Andreas M., 80804 Munich (DE); Bracher, Andreas., 80336 München (DE)
(74) Representative: Arth, Hans-Lothar

(57) **Abstract**

The present invention is directed to a FKBP subtype-specific rapamycin analogue selective for a single FKBP, wherein the FKBP subtype-specific rapamycin analogue preferentially forms a complex with only a single FKBP and this complex binds and/or inhibits the mammalian target of rapamycin for use in treatment of cancer, depression, neurodegeneration, transplant rejection or metabolic disorders.

## Description

### Field of the invention

The present invention is directed to a FKBP subtype-specific rapamycin analogue, wherein the FKBP subtype-specific rapamycin analogue preferably forms a complex with only a single FKBP and this complex binds and/or inhibits the mammalian target of rapamycin for use in treatment of cancer, depression, neurodegeneration, transplant rejection or metabolic disorders.

### Background of the invention

The kinase mTOR (mammalian Target of Rapamycin) integrates growth factor-dependent stimuli, amino acid availability and cellular energy levels to coordinate cell growth and proliferation. This PI3K-related kinase is part of at least two distinct multi-protein complexes (mTORC1 and mTORC2) that display different substrate specificities depending on the presence of the scaffolding proteins Raptor or Rictor. While Raptor recruits p70S6Kinase and 4E-BP1 as substrate for phosphorylation by mTORC1, Rictor mediates the activation of Akt by mTORC2 kinase activity. p70S6Kinase and 4E-BP1 are key regulators of the translation initiation complex and are therefore involved in the initiation of ribosomal protein biosynthesis. Akt is an important "survival" kinase that affects the expression of numerous genes involved in cell survival and cell cycle progression. Altogether, the PI3K/Akt/mTOR signalling pathway is crucial in regulating cell growth and protein metabolism.

mTORC1 can be inhibited by rapamycin, a small molecule originally isolated from the bacterium *S*. *hygroscopicus.* This compound is known to bind to FKBP12, and the resulting complex specifically interacts with the FRB (FK506-rapamycin binding) domain of mTOR, allosterically inhibiting the kinase activity. The FKBP12-rapamycin complex does not interact with mTORC2 and Akt kinase activation is not affected by acute rapamycin treatment. However, mTORC2 assembly seems to be rapamycin-sensitive in susceptible cell line upon long-term treatment. The molecular factors governing mTORC2 susceptibility are still unclear. Rapamycin is a FDA-approved drug used after organ transplantations that exerts its immunosuppressive action in humans by modulating T-cell proliferation. In recent years, rapamycin has also been found to successfully inhibit growth of certain cancer cell lines. Thus, it is used in chemotherapy, mainly in combination with other drugs (e.g., sorafenib). As the bio-availability of rapamycin itself is relatively poor, derivatives with optimised pharmacological properties have been developed ("rapalogs"). The compounds are approved or in clinical trials for treatment of various cancer types and have been shown to be effective especially in advanced renal cell carcinoma. Rapamycin has also been shown to prevent pathological protein aggregation or accumulation in animal models of Alzheimer's, Parkinson's and Huntington's disease which was attributed in part to its autophagy-promoting activity. In addition, rapamycin treatment was shown to prolong the life span of numerous organisms include mice. Unfortunately, the clinical use of rapamycin is generally restricted as the immunosuppression induced by rapamycin is a prohibitively severe side effect.

### Description of the Invention:

So far, the pharmacological effects of rapamycin are almost exclusively interpreted and discussed in the context of a complex with the prototypical FKBP12. However, direct protein-protein contacts in the FKBP12-rapamycin-FRB complex are very limited and their relevance is unknown.

Surprisingly, it was found that larger FKBP-rapamycin complexes potently bind and inhibit mTOR *in vitro* and *in vivo.* The inventors found that larger FKBP-rapamycin complexes almost equipotently inhibit mTOR. This opens the possibility for use of FKBP subtype-specific rapamycin analogues, something which has not been considered before. Thus far tissue-specific mTOR inhibition was not possible due to the ubiquitous nature of mTOR. Hence, by using FKBP subtype-specific mTOR inhibitors, tissue specific inhibition of mTOR can be achieved. This has possible beneficial implications regarding efficacy and especially reduced side effects.

Consequently, the present invention is directed to a FKBP subtype-specific rapamycin analogue selective for a single FKBP, wherein the FKBP subtype-specific rapamycin analogue preferably forms a complex with a single FKBP selected from the subtypes FKBP12, FKBP12.6, FKBP13, FKBP19, FKBP22, FKBP23, FKBP25, FKBP36 FKBP38, FKBP51, FKBP52, FKBP60, FKBP65 and FKBP133, wherein the complex binds to the mammalian target of rapamycin for use in treatment and/or prophylaxis of a disease selected from transplantation/allograft rejection, mTOR-dependent cancers, restenosis, diabetes, insulin resistance, obesity, metabolic syndrome, autoimmune diseases, tuberous sclerosis, depression, cognition and neurological disorder or for supporting cellular immune therapy.

In one embodiment the autoimmune disease is selected from systemic lupus erythematosus, Sjogren's syndrome and/or rheumatoid arthritis.

In another embodiment the expression "mTOR-dependent cancers" refers to cancers, wherein the activity of mTOR plays a major role in the occurrence/progression of said cancer and comprises cancers selected from :
adenocarcinoma, choroidal melanoma, acute leukemia, acoustic neurinoma, ampullary carcinoma, anal carcinoma, astrocytoma, basal cell carcinoma, pancreatic cancer, desmoid tumor, bladder cancer, bronchial carcinoma, non-small cell lung cancer (NSCLC), breast cancer, Burkitt's lymphoma, corpus cancer, CUP-syndrome (carcinoma of unknown primary), colorectal cancer, small intestine cancer, small intestinal tumors, ovarian cancer, endometrial carcinoma, ependymoma, epithelial cancer types, Ewing's tumors, gastrointestinal tumors, gastric cancer, gallbladder cancer, gall bladder carcinomas, uterine cancer, cervical cancer, cervix, glioblastomas, gynecologic tumors, ear, nose and throat tumors, hematologic neoplasias, hairy cell leukemia, urethral cancer, skin cancer, skin testis cancer, brain tumors (gliomas), brain metastases, testicle cancer, hypophysis tumor, carcinoids, Kaposi's sarcoma, laryngeal cancer, germ cell tumor, bone cancer, colorectal carcinoma, head and neck tumors (tumors of the ear, nose and throat area), colon carcinoma, craniopharyngiomas, oral cancer (cancer in the mouth area and on lips), cancer of the central nervous system, liver cancer, liver metastases, leukemia, eyelid tumor, lung cancer, lymph node cancer (Hodgkin's/Non-Hodgkin's), lymphomas, stomach cancer, malignant melanoma, malignant neoplasia, malignant tumors gastrointestinal tract, breast carcinoma, rectal cancer, medulloblastomas, melanoma, meningiomas, Hodgkin's disease, mycosis fungoides, nasal cancer, neurinoma, neuroblastoma, kidney cancer, renal cell carcinomas, non-Hodgkin's lymphomas, oligodendroglioma, esophageal carcinoma, osteolytic carcinomas and osteoplastic carcinomas, osteosarcomas, ovarial carcinoma, pancreatic carcinoma, penile cancer, plasmocytoma, squamous cell carcinoma of the head and neck (SCCHN), prostate cancer, pharyngeal cancer, rectal carcinoma, retinoblastoma, vaginal cancer, thyroid carcinoma, Schneeberger disease, esophageal cancer, spinalioms, T-cell lymphoma (mycosis fungoides), thymoma, tube carcinoma, eye tumors, urethral cancer, urologic tumors, urothelial carcinoma, vulva cancer, soft tissue tumors, soft tissue sarcoma, Wilm's tumor, cervical carcinoma and tongue cancer.

In another embodiment neurodegeneration or neurological disorder refers to Parkinson's disease, Alzheimer's disease, Huntington's disease and/or amyotrophic lateral sclerosis.

The FKBP subtype-specific rapamycin analogue is especially useful in neuroprotection, namely for treatment and/or prophylaxis of epilepsy and/or traumatic brain injury.

In context of the present invention use of a FKBP subtype-specific rapamycin analogue for treatment of autoimmune diseases or transplant rejection may also be the use of FKBP subtype-specific rapamycin analogue in ex vivo expansion of human T-regulatory cells which are subsequently used for treatment of autoimmune diseases or transplant rejection.

In further embodiments the disease is selected from the group consisting of psychiatric disorder, neurological disorder, glucocorticoid hyposensitivity syndrome, alopecia, abnormally elevated intraocular pressure, macular degeneration, oxidative damage to eye tissues, vision disorder, memory impairment, promoting hair growth, stimulating neurite growth, wound healing, antiglaucomatous medications, improving vision, enhancing memory performance, prevention of multi-drug resistance, limiting or preventing hemorrhage and neovascularization.

In a further embodiment the present invention is directed to the use of a FKBP subtype-specific rapamycin analogue selective for a single FKBP, wherein the FKBP subtype-specific rapamycin analogue forms a complex preferentially with a single FKBP12, FKBP12.6, FKBP13, FKBP19, FKBP22, FKBP23, FKBP25, FKBP36 FKBP38, FKBP51, FKBP52, FKBP60, FKBP65 and FKBP133, wherein the complex binds to the mammalian target of rapamycin, for the manufacture of a pharmaceutical composition for treatment and/or prophylaxis of a disease selected from transplantation/allograft rejection, mTOR-dependent cancers, restenosis, diabetes, insulin resistance, obesity, metabolic syndrome, autoimmune diseases, tuberous sclerosis, depression, cognition and/or neurological disorder or for supporting cellular immune therapy.

In a preferred embodiment transplantation rejection refers to transplantation rejection of the heart, liver, kidney and/or lung.

In a further embodiment the FKBP subtype-specific rapamycin analogue is used for treatment and/or prophylaxis of an affective disorder, wherein the affective disorder is selected from the group consisting of depression, bipolar disorder, mania, substance induced mood disorder and seasonal affective disorder (SAD).

In yet another embodiment the FKBP subtype-specific rapamycin analogue is used for treatment and/or prophylaxis of an anxiety disorder, wherein the anxiety disorder is selected from the group comprising or consisting of generalized anxiety disorder, panic disorder, panic disorder with agoraphobia, phobias, obsessive-compulsive disorder, post-traumatic stress disorder, separation anxiety and childhood anxiety disorders.

Among the hundreds of different neurodegenerative disorders, the attention has been given only to a handful, including Alzheimer's Disease, Parkinson's Disease, and amyotrophic lateral sclerosis.

Among the glucocorticoid hyposensitivity syndromes, the attention has been given to the group of related diseases enclosing resistant asthma, AIDS, rheumatoid arthritis, hypertension and obesity.

Among the cancers, the attention has been given to malignant melanoma or prostate cancer.

Among the vision disorders, the attention has been given to visual impairments; orbital disorders; disorders of the lacrimal apparatus; disorders of the eyelids; disorders of the conjunctiva; disorders of the Cornea; cataract; disorders of the uveal tract; disorders of the retina; disorders of the optic nerve or visual pathways; free radical induced eye disorders and diseases; immunologically-mediated eye disorders and diseases; eye injuries; and symptoms and complications of eye disease, eye disorder, or eye injury.

FKBP subtypes are differentially expressed in cells and tissues that are responsive to desired and undesired effects of rapamycin *in vivo.* For the cytosolic FKBPs the contributions of the individual FKBP homologs might be dictated largely by their relative cellular abundance. A tissue-specific mTOR inhibition is extremely attractive because classical systemic rapamycin treatment causes potent immunosuppression which is highly beneficial for transplantation but unacceptable for most other potential indications. In one embodiment the FKBP subtype-specific rapamycin analogue is selected from the group consisting of: Biolimus A9, Zotarolimus, Everolimus, Myolimus, Novolimus, Pimecrolimus, Tacrolimus, Ridaforolimus and Temsirolimus.

In another embodiment the FKBP subtype-specific rapamycin analogue is selective for two FKBPs selected from the group consisting of: FKBP12, FKBP12.6, FKBP13, FKBP19, FKBP22, FKBP23, FKBP25, FKBP36 FKBP38, FKBP51, FKBP52, FKBP60, FKBP65 and FKBP133.

In yet another embodiment the FKBP subtype-specific rapamycin analogue is selective for three FKBPs selected from the group consisting of: FKBP12, FKBP12.6, FKBP13, FKBP19, FKBP22, FKBP23, FKBP25, FKBP36 FKBP38, FKBP51, FKBP52, FKBP60, FKBP65 and FKBP133.

In yet another embodiment the FKBP subtype-specific rapamycin analogue is selective for four FKBPs selected from the group consisting of: FKBP12, FKBP12.6, FKBP13, FKBP19, FKBP22, FKBP23, FKBP25, FKBP36 FKBP38, FKBP51, FKBP52, FKBP60, FKBP65 and FKBP133.

Preferentially the FKBP subtype-specific rapamycin analogue is selective for FKBP12 and FKBP52, preferentially FKBP12, FKBP12.6 and FKBP52, preferred FKBP12 and FKBP51, preferred FKBP12, FKPB51 and FKBP52, further preferred FKBP12, FKBP12.6, FKBP51 and FKBP52, preferably FKBP51 and FKBP52, preferred FKBP12 and FKBP38, preferably FKBP12, FKBP38 and FKBP51, preferably FKBP12, FKBP38, FKBP51 and FKBP52, preferably FKBP12, FKBP38 and FKBP52, preferably FKBP12, FKBP12.6 and FKBP38, preferably FKBP12, FKBP12.6, FKBP38 and FKBP51, preferably FKBP12, FKBP12.6, FKBP38 and FKBP52, preferably FKBP12.6 and FKBP38, preferably FKBP12.6, FKBP38 and FKBP51, preferably FKBP12.6 and FKBP51, preferably FKBP12.6 and FKBP52, preferably FKBP12.6 and FKBP12, preferably FKBP38 and FKBP51, preferably FKBP38 and FKBP52, preferably FKBP12 and FKBP13, preferably FKBP12 and FKBP25, preferably FKBP12.6 and FKBP13, preferably FKBP12.6 and FKBP25, preferably FKBP38 and FKBP13, preferably FKBP38 and FKBP25, preferably FKBP51 and FKBP13, preferably FKBP51 and FKBP25, preferably FKBP52 and FKBP13, preferably FKBP52and FKBP25, preferably FKBP12, FBKP12.6 and FKBP13, preferably FKBP12, FBKP12.6 and FKBP25, preferably FKBP12, FKBP38 and FKBP13, preferably FKBP12, FBKP38 and FKBP25, preferably FKBP12, FKBP51and FKBP13, preferably FKBP12, FKBP51and FKBP25, preferably FKBP12, FKBP52and FKBP13, preferably FKBP12, FKBP52and FKBP25, preferably FKBP12, FKBP12.6, FKBP13 and FKBP25, preferably FKBP12, FKBP38, FKBP13 and FKBP25, preferably FKBP12, FKBP51, FKBP13 and FKBP25, preferably FKBP12, FKBP52, FKBP13 and FKBP25, preferably FKBP38, FKBP12.6, FKBP13 and FKBP25, preferably FKBP51, FKBP12.6, FKBP13 and FKBP25, preferably FKBP52, FKBP12.6, FKBP13 and FKBP25, preferably FKBP38, FKBP51, FKBP13 and FKBP25, preferably FKBP38, FKBP52, FKBP13 and FKBP25.

The following formulas are examples for rapamycin analogues according to claim 1 of the present invention The mentioned rapamycin analogues belong to the group consisting of:

Compounds of the formula according to claim 1 of the reference EP0467606A1 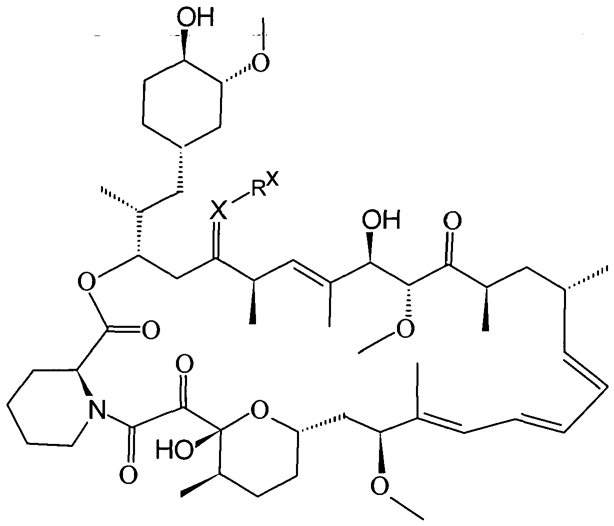 wherein
**R^{x}** represents -N**R¹R²** or -O**R³** ; **R¹** is hydrogen, alkyl of 1-6 carbon atoms, or aralkyl of 7-10 carbon atoms; **R²** is hydrogen, alkyl of 1-6 carbon atoms, cycloalkyl of 3-10 carbon atoms, aralkyl of 7-10 carbon atoms, -CO**R⁴**, -C0₂**R⁴**, - S0₂**R⁴**, -CON**R⁵R⁶**, -CSN**R⁵R⁶**, -CSN**R⁵R⁶**, -COCON**R⁵R⁶**, or **A**; **R³** is hydrogen, alkyl of 1-6 carbon atoms or -CH₂**A**; **R⁴** is alkyl of 1-6 carbon atoms, aralkyl of 7-10 carbon atoms, or **A** ; **R⁵** and **R⁶** are each, independently, hydrogen, alkyl of 1 to 6 carbon atoms, cycloalkyl of 3-10 carbon atoms, allyl, aralkyl of 7-10 carbon atoms, or **A**;
**A** represents 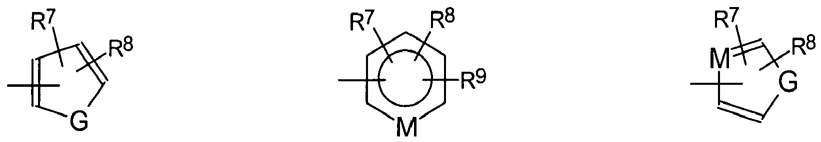 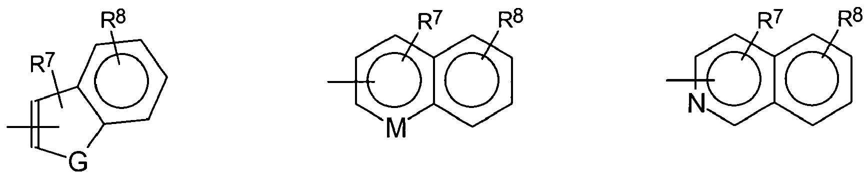 **M** represents -CH- or N, **G** represents NH, O, S;
**R⁷**, **R⁸**, **R⁹** are each, independently, hydrogen, alkyl, alkoxy, hydroxy, cyano, halo, nitro, carbalkoxy, trifluoromethyl, trifluoromethoxy, amino, or a carboxylic acid.

Compounds of the formula according to claim 1 of the reference EP0754040A1 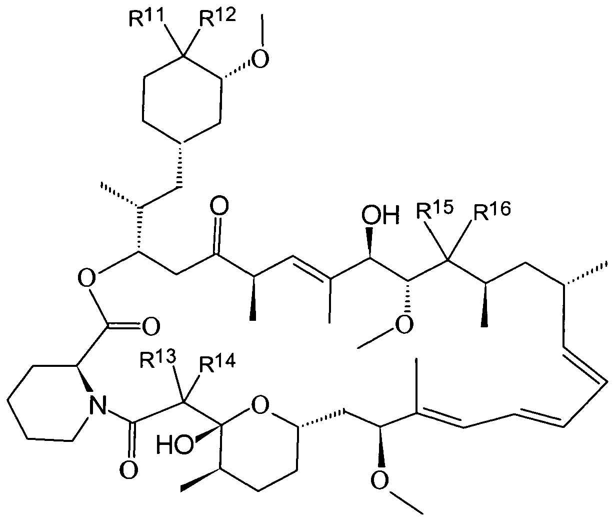 **R¹¹** and **R¹²** are selected from the group consisting of =O, (-OH,H) and (H,H);
**R¹³** and **R¹⁴** are selected from the group consisting of =O, (H,H) and (H,OH); and
**R¹⁵** and **R¹⁶** are selected from the group consisting of =O and (H,OH).

Compounds of the formula according to claim 1 of the reference EP1785430A1 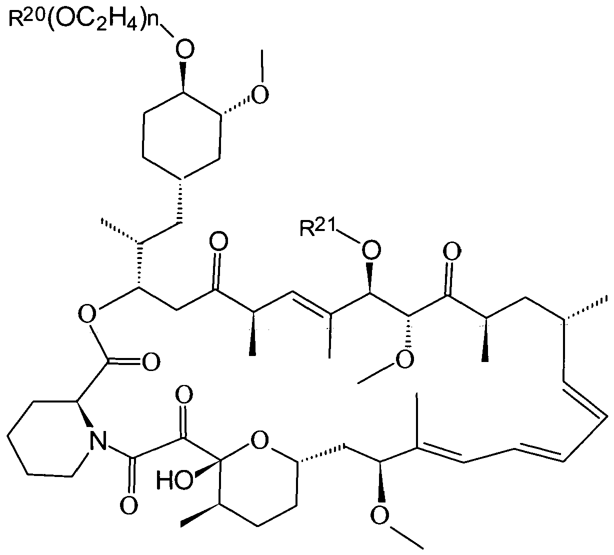 wherein
n = 0 or 1, **R²⁰** and **R²¹** are independently hydrogen or **-Y-Z**, wherein each **Y** is a linker, and each **Z** is a carbohydrate moiety independently selected from the group consisting of a monosaccharide, an oligosaccharide and a pseudosugar wherein **Z** is attached to **Y** through a hydroxyl oxygen atom of **Z** with the proviso that **R²⁰** and **R²¹** are not both hydrogen;

In an embodiment, **R²⁰** can be hydrogen and **R²¹** can be **Y-Z** or in another embodiment, **R²¹** is hydrogen and **R²⁰** is **Y-Z;** In a further embodiment, **Y** can be selected from the group consisting of (i) -**R²²**C(0)-; (ii) -C(0)**R²²**-; iii) -**R²²**S(0)₂-; and (iv) -S(0)₂**R²²**-; wherein **R²²** is selected from the group consisting of: (a) - (CH₂)p- where p is an integer from 1 to 18; (b) -(CH₂)ₙ-0-(CH₂)ₘ- where n and m are each independently an integer from 2 to 6; and (c) a bond; in another embodiment, **Y** can be selected from the group consisting of -C(O)- and - SO₂-; In an additional embodiment, **Y** can be a single functional group; in another aspect, **Z** can be selected from the group consisting of fructose, fucitol and allose; in yet a further aspect, **Z** can be a monosaccharide derivative wherein at least one of the hydroxyl groups of the monosaccharide is replaced with a hydrogen, an alkoxy, an alkanoate or a halogen group.

Compounds of the formula according to claim 1 of the reference US5912253A, US6200985B1 and US5985890A 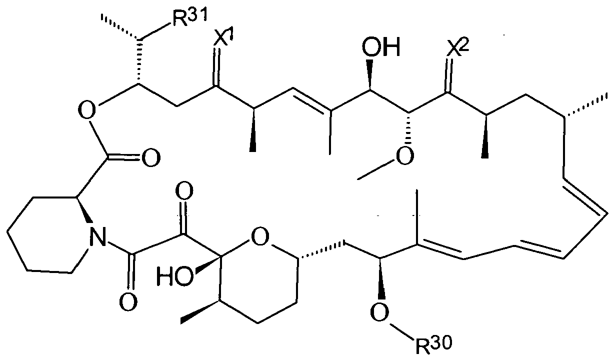 or 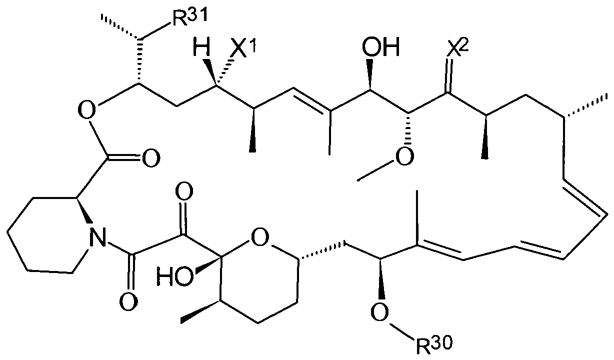 wherein **R³⁰** is: optionally hydroxy-substituted alkynyl;
**R³¹** is selected from: 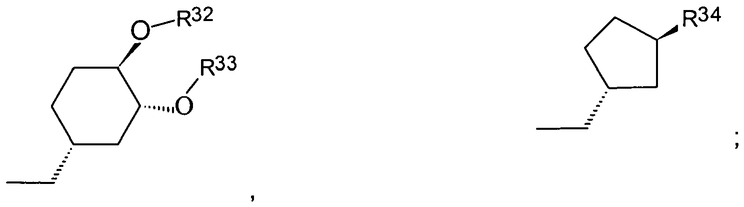 **R³²** is: H, alkyl, alkyenyl, (optionally hydroxy-substituted) alkynyl, aryl, thioalkyl, arylalkyl, hydroxyarylalkyl, hydroxyaryl, hydroxyalkyl, dihydroxyalkyl, hydroxyalkoxyalkyl, hydroxyalkylarylalkyl, dihydroxyalkylarylalkyl, alkoxyalkyl, aminoalkyl, alkylaminoalkyl, alkoxycarbonylamidoalkyl, arylsulfonamidoalkyl, allyl, dihydroxyalkylallyl, dioxolanylallyl, carbalkoxyalkyl, or alkylsilyl;
**R³³** is H or methyl, or **R³³** together with **R³²** forms C2-6 alkylene;
**R³⁴** is: **R³⁵**-O-CH₂- wherein **R³⁵** is H, alkyl, alkenyl, alkynyl, aryl, amino, alkylcarbonyl, arylcarbonyl, heteroarylcarbonyl, hydroxyalkylcarbonyl, aminoalkylcarbinyl, formyl, thioalkyl, arylalkyl, hydroxyarylalkyl, hydroxyaryl, hydroxyalkyl, dihydroxyalkyl, hydroxyalkoxyalkyl, hydroxyalkylarylalkyl, dihydroxyalkylarylalkyl, alkoxyalkyl, aminoalkyl, alkylaminoalkyl, alkoxycarbonylamidoalkyl, arylsulfonamidoalkyl, allyl, dihydroxyalkylallyl, dioxolanylallyl, carbalkoxyalkyl or alkylsilyl, **R³⁶** CO- where **R³⁶** is H, alkyl, hydroxy, alkoxy, aryloxy, amido, alkamido, a residue of an amino acid, or N,N-disubstituted-amido wherein each substituent of N is independently alkyl, aryl, arylakyl or alkylaryl, or both such substituents together form morpholino or piperazino, **R³⁷** NCH- where **R³⁷** is alkyl, aryl, amino, alkylamino, arylamino, or arylsulfonylamino, or O,O-(alkylyne) dioxymethylyne; each **X¹** and **X²** is independently O, (H, OH), or (H, O**R³⁸**); and **R³⁸** is: alkyl, alkylcarbonyl, arylcarbonyl, heteroarylcarbonyl, hydroxyalkylcarbonyl, aminoalkylcarbonyl, formyl or aryl; "alk" or "alkyl" refer to a C1-10 aliphatic substituent, each such "alk" or "alkyl" being optionally interrupted by an oxy (-O-) linkage; and "ar" or "aryl" refer to substituted or unsubstituted tolyl, phenyl, benzyl, or pyridyl. Compounds of the formula according to claim 1 of the reference WO9802441 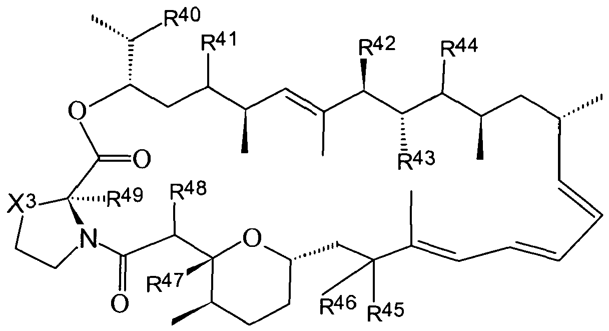 wherein **R⁴⁰** is: 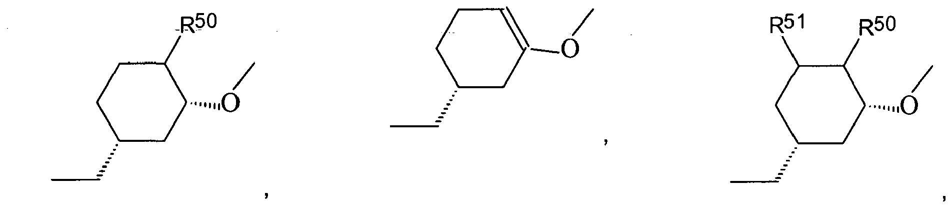 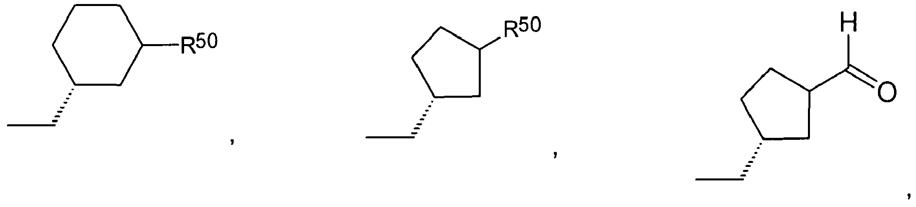 one of **R⁴⁶** and **R⁴⁵** is H and the other is -H, halo, -**R⁵³**, -O**R⁵²**, -S**R⁵²**, -OC(O)**R⁵²**,-OC(O)NH**R⁵²**, -NH**R⁵²**, -NH**R⁵²R⁵³**, -NHC(O)**R⁵²**, or -NH-SO2-**R⁵²**, where **R⁵³** = aliphatic, heteroaliphatic, aryl, heteroaryl or alkylaryl;
**R⁴⁴** is halo, -O**R⁵⁴** or (=O),
**R⁴¹** is =O, =N**R**^{**5**5} =NO**R⁵⁵** or =NNH**R⁵⁵**, -NHO**R⁵⁵** or -NHNH**R⁵⁵**, -O**R⁵⁵**, -OC(O)**R⁵⁵** - OC(O)N**R⁵⁵**, halo or -H,
**R⁴⁸** is =O, -O**R⁵⁷**, -N**R⁵⁷**, -H, -NC(O)**R⁵⁷**, -OC(O)**R⁵⁷** or -OC(O)N**R⁵⁷**;
**R⁵⁴** is H, -**R⁵⁸**, -C(O)**R⁵⁸** or -C(O)NH**R⁵⁸** or a cyclic moiety bridging C28 and C30;
**R⁴²** is halo or -O**R⁵⁴**;
**R⁴³** is H, OH or OMe;
**R⁴⁹** is **R⁵⁵**;
where each substituent is present in either stereochemical orientation unless otherwise indicated, and where **R⁵²**, **R⁵⁵**, **R⁵⁶**, **R⁵⁷**, **R⁵⁸**, **R⁶⁰**, **R⁶¹** and **R⁶²** are independently selected from H, aliphatic, heteroaliphatic, aryl or heteroaryl;
**R⁵⁹** is H, halo, -CN, =O, -OH, -N**R⁶⁰R⁶¹**, OS02CF3, OSO2F, OSO2**R⁵⁵**', OCO**R⁵⁵**', OCON**R⁵⁵_{'}R⁵⁶'**, or OCON(O**R⁵⁵**')**R⁵⁶**';
in which one or both of **R⁴⁷** and **R⁴²** is a halo substituent; both **R⁴¹** and **R⁴⁴** are other than =O; one of **R⁴⁶** and **R⁴⁷** is H and the other is phenyl, di- or trisubstituted phenyl or a mono- or di-substituted heterocyclic moiety; **X³** is -CH₂- or -CH₂-CH₂-; and/or moiety **R⁴⁰** is other than 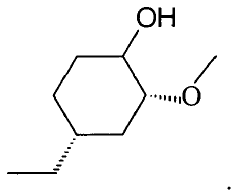 Compounds of the formula according to claim 1 of the reference US2008021211A1 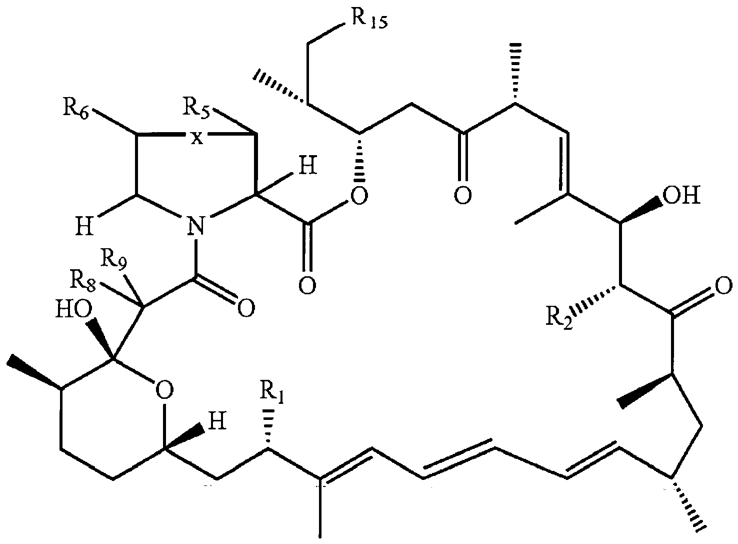 where:
x=bond or CH₂, or -CH**R⁶**-x-CH**R⁵**- is 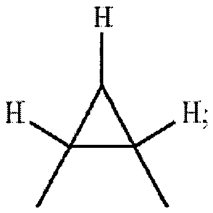
**R¹**= OH, OCH₃;
**R²**= OCH₃;
**R⁵**= H, OH;
**R⁶**= H, OH;
**R⁸** and **R⁹** together are -O or H,H;
**R¹⁵** is 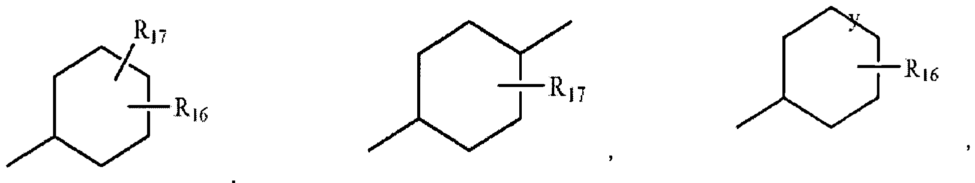 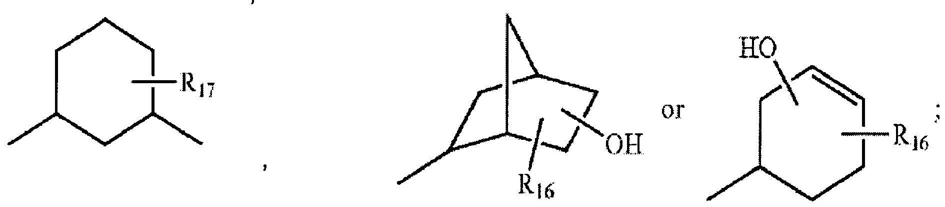 wherein **R¹⁶ R¹⁷** = OCH₃, H, OH, Cl, F.

Compounds of the formula according to claim 1 of the reference US2008004264A1 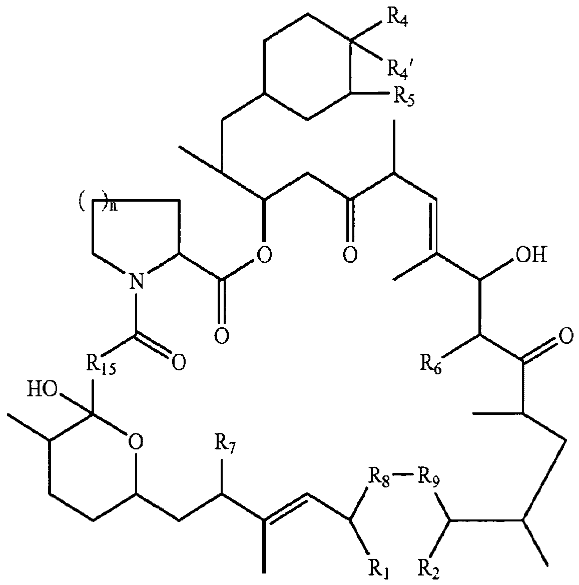 wherein:
**R¹** and **R²** are different, independent groups and are selected from the group consisting of O**R³** and N(**R³**')(**R³**");
or **R¹** and **R²** are different, are connected through a single bond, and are selected from the group consisting of O and N**R³**; **R³**, **R³'**, and **R³"** are independently selected from the group consisting of H, C1 to C6 alkyl, C1 to C6 substituted allyl, C3 to C8 cycloalkyl, substituted C3 to C8 cycloalkyl, aryl, substituted aryl, heteroaryl, and substituted heteroaryl;
**R⁴** and **R⁴'** are: (a) independently selected from the group consisting of H, OH, O(C1 to C6 alkyl), O(substituted C1 to C6 alkyl), O(acyl), O(aryl), O(substituted aryl), and halogen; or (b) taken together to form a double bond to O;
**R⁵, R⁶**, and **R⁷** are independently selected from the group consisting of H, OH, and OCH₃;
**R⁸** and **R⁹** are connected through a single bond and are CH₂; **R¹⁵** is selected from the group consisting of C-O, CHOH, and CH₂; n is 1 or 2.

Compounds of the formula according to claim 1 of the reference US2010061994A1 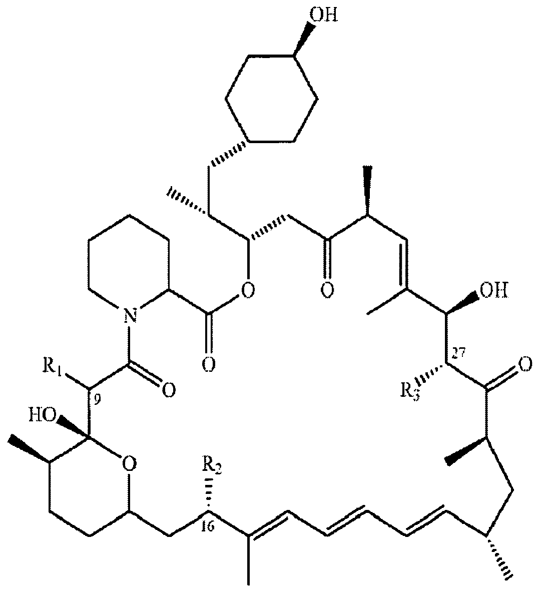 wherein, **R¹** represents (H,H) or -O and **R²** and **R³** each independently represent H, OH or OCH₃,

Compounds of the formula according to claim 1 of the reference US2003114477A1 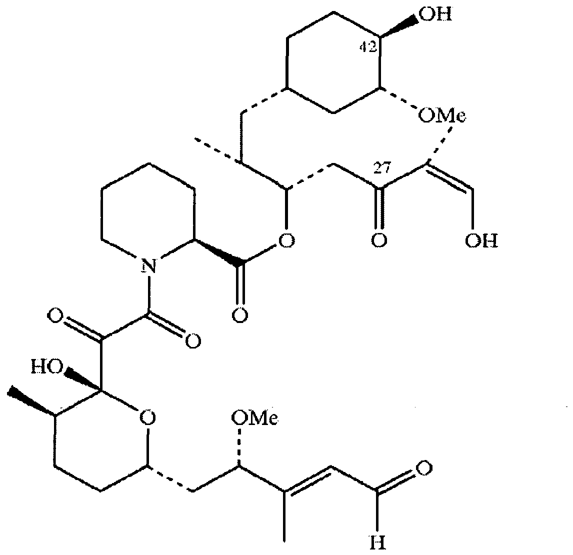 or

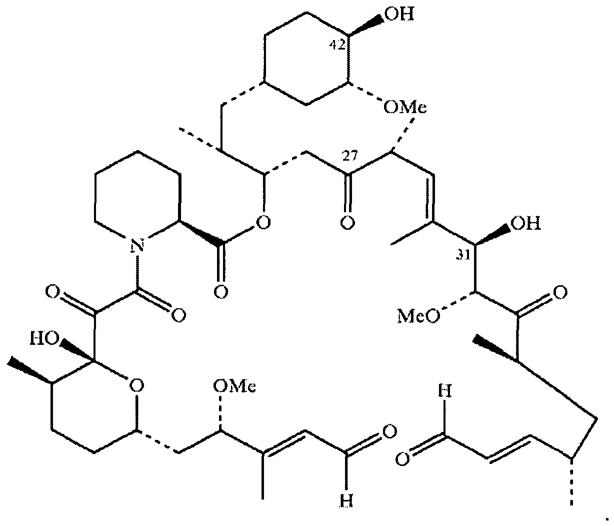

Compounds of the formula according to claim 1 of the reference WO2000009109 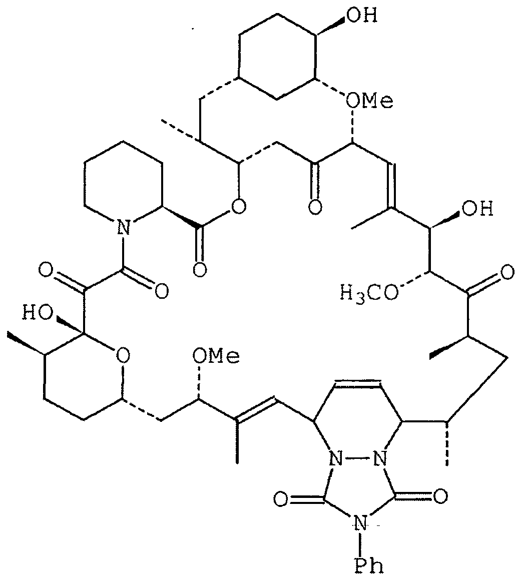 or 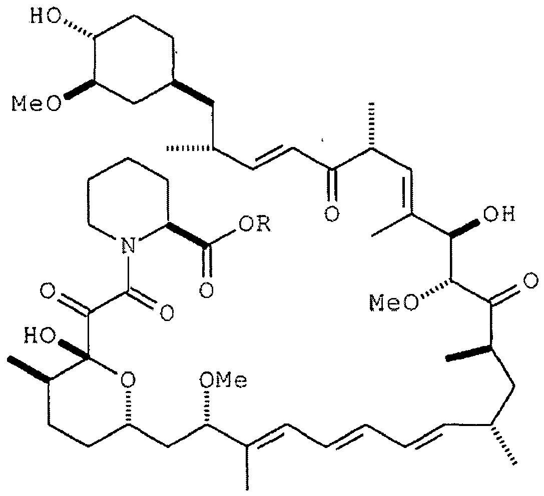 **R** represents -Me, -CH₂-Ph;
or 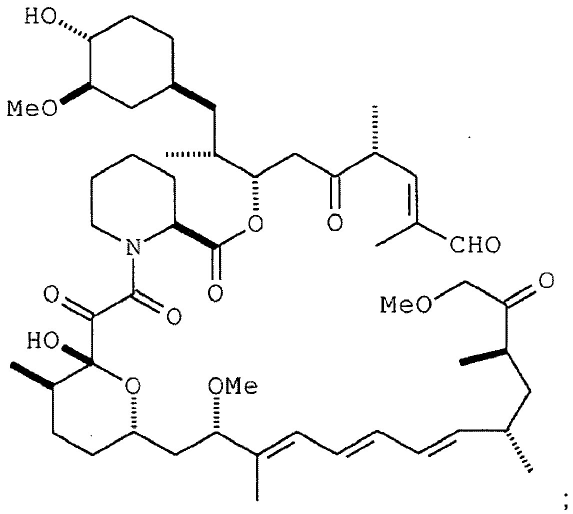 or

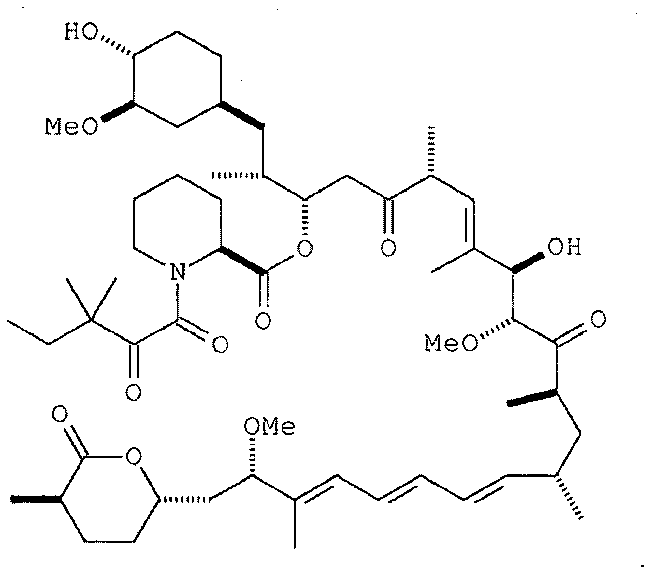

Compounds of the formula according to claim 1 of the reference EP1571151 B1 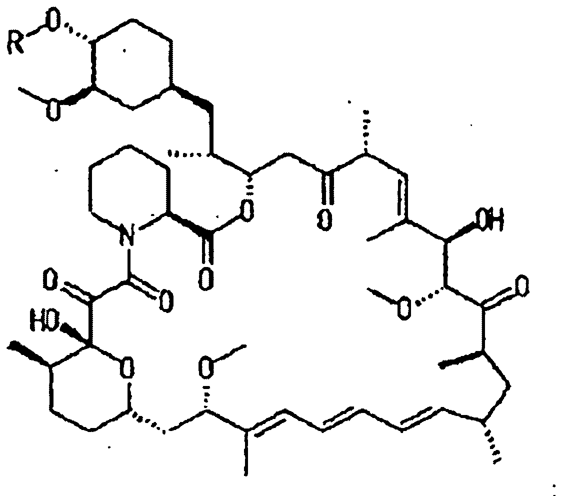 where **R** denotes alkyl, arylalkyl, hydroxyalkyl, alkoxyalkyl, acyloxyalkyl, aminoalkyl, alkylaminoalkyl, alkoxycarbonylaminoalkyl or acylaminoalkyl.

Compounds of the formula according to claim 1 of the reference JP2007231015A 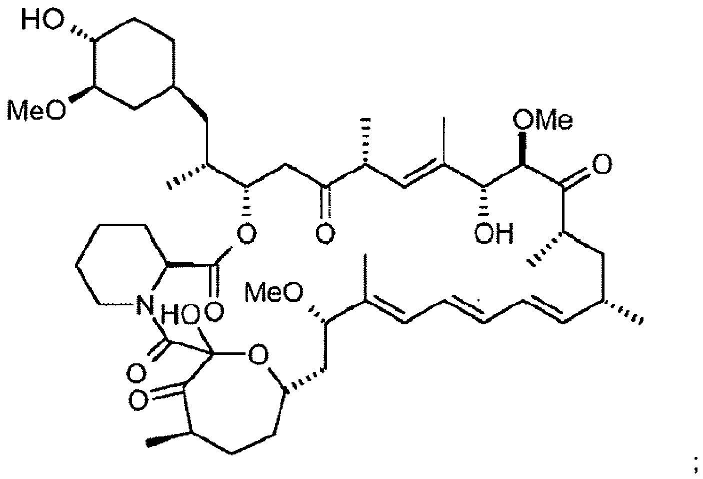 or

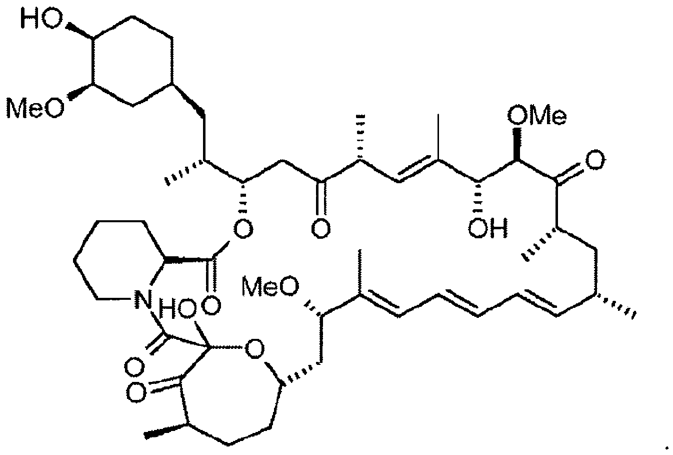

Compounds of the formula according to claim 1 of the reference MX2008010804A 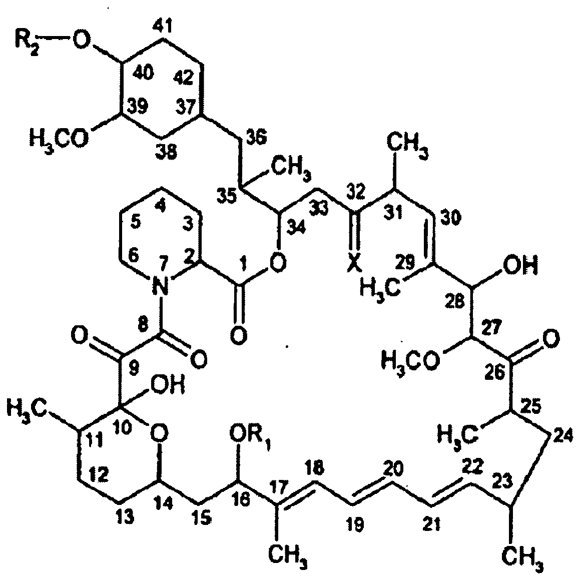 wherein **R₁** represents -CH₃ or an alkyl chain of 3 to 6 carbon,
**R₂** represents -H, -CH₂-CH₂-OH o -CH₂-CH₂-O-alkyl (1 to 8 carbon atoms), -CH₂-CH₂-O-CH₂-CH₃, and **X** represents O, (H, H) or (H, OH).

Compounds of the formula according to claim 1 of the reference US5310903A 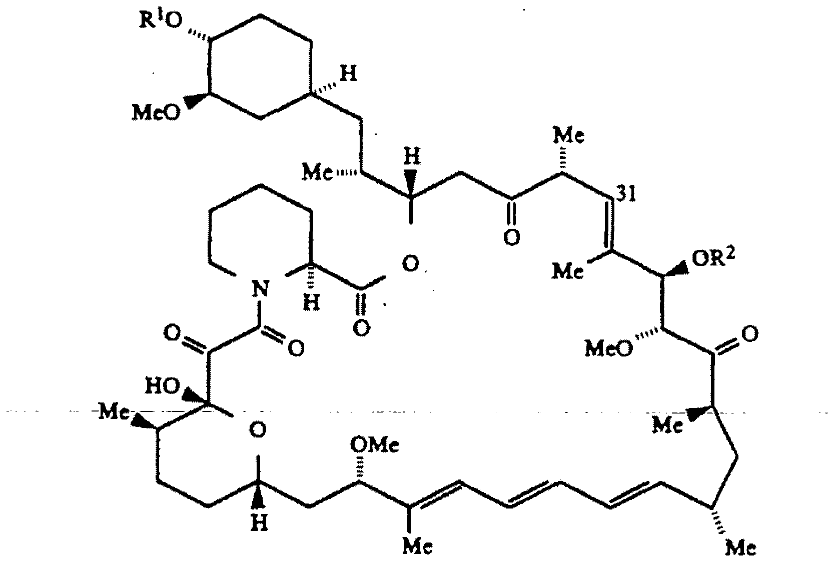 wherein: **R¹** is selected from the group consisting of: 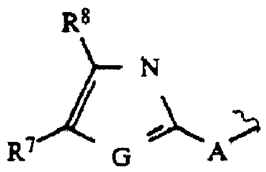 wherein **G** is N-**R⁶**, O, S, SO, or SO₂,

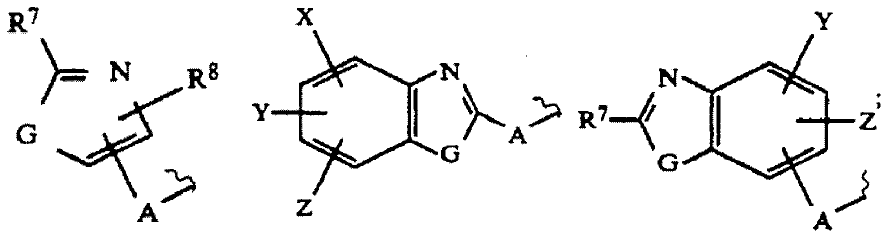

**R²** is independently selected from:
(1) the definitions of **R¹** ;
(2) hydrogen;
(3) phenyl;
(4) substituted phenyl in which the substituents are **X**, **Y** and **Z**;
(5) 1- or 2-naphthyl;
(6) substituted 1- or 2-naphthyl in which the substituents are **X**, **Y** and **Z**;
(7) biphenyl;
(8) substituted biphenyl in which the substituents are **X**, **Y** and **Z**;
(9) C1-10 alkyl;
(10) substituted C1-10 alkyl in which one or more substituent(s) is(are) selected from:
   (a) hydroxy, (b) oxo, (c) C1-6 -alkoxy, (d) phenyl-C1-3 alkoxy, (e) substituted phenyl-C1-3 -alkoxy, in which the substituents on phenyl are **X**, **Y** and **Z**, (f) - OCO-C1-6 alkyl, (g) -N**R⁹ R¹⁰**, wherein **R⁹** and **R¹⁰** are independently selected from: (i) hydrogen, or (ii) C1-6 alkyl unsubstituted or substituted with one or more of the substituent(s) selected from: (a') phenyl, which may be substituted with **X**, **Y** and **Z**, (b') -OH, (c') C1-6 alkoxy, (d') -CO₂H, (e') -CO₂-C1-6 alkyl, (f) -C3-7 cycloalkyl, and (g') -O**R¹¹**, (iii) or where **R⁹** and **R¹⁰** and the N to which they are attached may form a heterocyclic ring selected from the group consisting of: morpholine, thiomorpholine, piperidine, and piperazine, (h)-N**R⁹** CO-C1-6 alkyl-**R¹⁰**, wherein **R⁹** and **R¹⁰** are as defined above, (i) -COO**R⁹**, wherein **R⁹** is as defined above, (j) -CHO, (k) phenyl, (I) substituted phenyl in which the substituents are **X**, **Y** and **Z**, (m) 1- or 2-napthyl, (n) substituted 1- or 2-naphthyl in which the substituents are **X**, **Y** and **Z**, (o) biphenyl, (p) substituted biphenyl in which the substituents are **X**, **Y** and **Z**, (q) -O**R¹¹**, and (r) -S(O)ₚ -C1-6 alkyl;
(11) C3-10 alkenyl;
(12) substituted C3-10 alkenyl in which one or more substituent(s) is(are) selected from:
   (a) hydroxy, (b) oxo, (c) C1-6 -alkoxy; (d) phenyl-C1-3 alkoxy; (e) substituted phenyl-C1-3 alkoxy, in which the substituents on phenyl are **X**, **Y** and **Z**, (f) - OCO-C1-6 alkyl, (g) -N**R⁹ R¹⁰**, wherein **R⁹** and **R¹⁰** are as defined above, (h) - N**R⁹**-CO-C1-6 alkyl, wherein **R⁹** is as defined above, (i) -COO**R⁹**, wherein **R⁹** is as defined above, (j) -CHO, (k) phenyl, (I) substituted phenyl in which the substituents are **X**, **Y** and **Z**, (m) 1- or 2-naphthyl, (n) substituted 1- or 2-naphthyl in which the substituents are **X**, **Y** and **Z**, (o) biphenyl, (p) substituted biphenyl in which the substituents are **X**, **Y** and **Z**; (q) -O**R¹¹**, and (r) -S(O)ₚ-C1-6 alkyl;
(13) C3-10 alkynyl;
(14) substituted C3-10 alkynyl in which one or more substituent(s) is(are) selected from:
   (a) hydroxy, (b) oxo, (c) C1-6 alkoxy, (d) phenyl-C1-3 alkoxy, (e) substituted phenyl-C1-3 -alkoxy, in which the substituents on phenyl are **X**, **Y** and **Z**, (f) - OCO-C1-6 alkyl, (g) -N9 **R¹⁰**, wherein **R⁹** and **R¹⁰** are as defined above, (h) - N**R**⁹-CO-C1-6 -alkyl, wherein **R⁹** is as defined above, (i) -COO**R⁹**, wherein **R⁹** is as defined above, (j) -CHO, (k) phenyl, (I) substituted phenyl in which the substituents are **X**, **Y** and **Z**, (m) 1- or 2-naphthyl, (n) substituted 1- or 2-naphthyl in which the substituents are **X**, **Y** and **Z**, (o) biphenyl, (p) substituted biphenyl in which the substituents are **X**, **Y** and **Z**, (q) -O**R¹¹** ; and
(15) -**R¹¹** ;

**R⁶** is selected from the group consisting of:
(1) hydrogen,
(2) C1-6 -alkyl, unsubstituted or substituted with:
   (a) hydroxy, (b) C1-6 alkoxy, (c) -**R¹²** -**R¹³**, wherein **R¹²** and **R¹³** are independently selected from (i) hydrogen, (ii) C1-6 alkyl, or (iii) C3-6 alkenyl,
   (d) phenyl, unsubstituted or substituted with **X**, **Y** and **Z**, (e) -O**R¹¹**,
(3) C3-6 alkenyl, unsubstituted or substituted with: (a) hydroxy, (b) phenyl, unsubstituted or substituted with **X**, **Y** and **Z**, or (c) C1-6 alkoxy,
(4) phenyl, unsubstituted or substituted with **X**, **Y** and **Z**,
(5) -**R¹¹**,
(6) **X**, **Y** or **Z**;

**R⁷** and **R⁸** independently are selected from the group consisting of:
(1) hydrogen,
(2) C1-7 alkyl,
(3) C2-6 alkenyl,
(4) -(CH₂)ₘ-N**R⁹ R¹⁰**, wherein **R⁹** and **R¹⁰** are as defined above, and m is 0, 1, 2, and 3,
(5) -CF₃,
(6) -CON**R⁹ R¹⁰**, wherein **R⁹** and **R¹⁰** are as defined above,
(7) **R¹⁴** O(CH₂)ₘ - wherein **R¹⁴** is hydrogen, C1-6 alkyl, hydroxy-C2-3 alkyl, -CF₃, phenyl, **R¹¹** or naphthyl and m is as defined above,
(8) 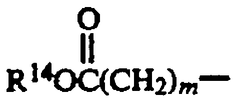 wherein **R¹⁴** and m are as defined above;
(9) phenyl-(CH₂)ₘ - wherein m is as defined above and the phenyl is unsubstituted or substituted with **X**, **Y** and **Z**,
(10) napthyl-(CH₂)ₘ - wherein m is as defined above and the napthyl is unsubstituted or substituted with **X**, **Y** and **Z**,
(11) biphenyl-(CH₂)ₘ - wherein m is as defined above and the biphenyl is unsubstituted or substituted with **X**, **Y** and **Z**,
(12) heteroaryl-(CH₂)ₘ - wherein heteroaryl is selected from: acridine, carbazole, cinnoline, dibenzofuran, dibenzothiophene, quinozaline, pyrrazole, indole, imidazole, benzatriazole, furan, benzofuran, quinoline, isoquinoline, pyrazine, pyridazine, pyridine, pyrimidine, and pyrrole, m is as defined above and the heteroaryl is unsubstituted or substituted with **X**, **Y**, and **Z**,
(13) morpholinyl, and
(14) -CH=CH-phenyl wherein the phenyl is unsubstituted or substituted with **X**, **Y** and **Z**;

**R¹¹** is selected from:
(a) -PO(OH)O- M⁺, wherein M⁺ is a positively charged inorganic or organic counterion, selected from the group consisting of: ammonium, sodium, lithium, potassium, calcium, magnesium, dicyclohexylamine, N-methyl-D-glucamine, arginine and lysine,
(b) -SO₃⁻ M⁺,
(c) -CO(CH₂)_{q} CO₂⁻ M⁺, wherein q is 1 to 3, and
(d) -CO-C1-6 alkyl-N**R⁹ R¹⁰**, wherein **R⁹** and **R¹⁰** are as defined above and the alkyl is unsubstituted or substituted with one or more substituents selected from:
   (i) hydrogen, (ii) C1-6 alkoxy, (iii) -N**R¹² R¹³**, wherein **R¹²** and **R¹³** are as defined above, (iv) -COO**R⁶**, wherein **R⁶** is as defined above, (v) phenyl, (vi) substituted phenyl in which the substituents are **X**, **Y** and **Z**, (vii) imidazolidyl, (viii) indolyl, (ix) -SH, and (x) -S-C1-6 alkyl;

**A** is selected from the group consisting of:
(1) a bond,
(2) C1-10 alkyl;
(3) substituted C1-10 alkyl in which one or more substituent(s) is(are) selected from:
   (a) hydroxy, (b) oxo, (c) C1-6 alkoxy, (d) phenyl-C1-3 alkoxy, (e) substituted phenyl-C1-3 alkoxy, in which the substituents on phenyl are **X**, **Y** and **Z**, (f) -OCO-C1-6 alkyl, (g) -N**R⁹ R¹⁰**, wherein **R⁹** and **R¹⁰** are as defined above, (h) -N**R⁹** CO-C1-6 alkyl, wherein **R⁹** is as defined above, (i) -COO**R⁹**, wherein **R⁹** is as defined above, (j) -CHO, (k) phenyl, (I ) substituted phenyl in which the substituents are **X**, **Y** and **Z**, (m) 1- or 2-naphthyl, (n) substituted 1- or 2-naphthyl in which the substituents are **X**, **Y** and **Z**, (o) biphenyl, (p) substituted biphenyl in which the substituents are **X**, **Y** and **Z**, (q) -O**R¹¹**, and (r) -S(O)ₚ -C1-6 alkyl;
(4) -C1-10 alkyl wherein one or more of the alkyl carbons is replaced by a group selected from: -N**R⁹** -, -O-, -S(O)ₚ -, -CO₂ -, -O₂C-, -CON**R⁹** -, -N**R⁹** CO-, -N**R⁹** CON**R¹⁰** -;
(5) -C3-10 alkenyl wherein alkenyl contains one to four double bonds and wherein one or more of the alkyl carbons is replaced by a group selected from: -N**R**⁹ -, -O-, -S(O)ₙ -, -CO₂C-, -CON**R⁹** -, -N**R⁹** CO-, and -N**R⁹** CON**R¹⁰** -; 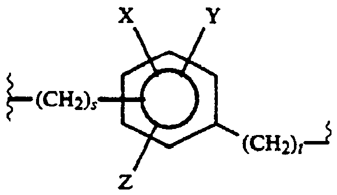 wherein s is 0 to 6 and t is 0 to 6, 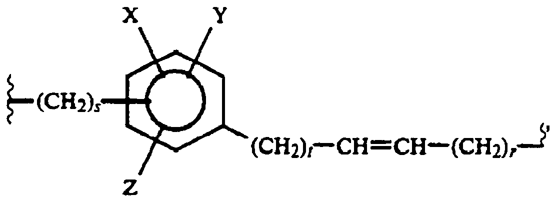 wherein r is 1 to 3 and s, and t are as defined above; **X**, **Y** and **Z** are independently selected from the group consisting of:
   (a) hydrogen,
   (b) C1-10 alkyl, unsubstituted or substituted with one or more substituents selected from:
      (i) aryl, wherein aryl is selected from the group consisting of phenyl and naphthyl, (ii) substituted aryl wherein aryl is as defined above and in which the substituents are **X'**, **Y'** and **Z'**, (iii) heteroaryl, wherein heteroaryl is as defined above, (iv) substituted heteroaryl wherein heteroaryl is as defined above in which the substituents are **X'**, **Y'**, and **Z'**, (v) unsubstituted or substituted aryloxy, wherein aryl is as defined above and in which the substituents on aryl are **X'**, **Y'** and **Z'**, (vi) -O**R⁹**, (vii) -O**R¹¹**, (viii) -OCO**R⁹**, (ix) -OCO2 **R⁹**, (x) -N**R⁹ R¹⁰**, (xi) -CHO, (xii) -NR⁹ COC1-6 alkyl-**R¹⁰**, (xiii) -N**R⁹** CO₂ C1-6 alkyl-**R¹**0, (xiv) -N**R⁹** CON**R⁹ R¹⁰**, (xv) - OCON**R⁹ R¹⁰**, (xvi) -CON**R⁹ R¹⁰**,
   (c) C1-10 alkyl wherein one or more of the alkyl carbons is replaced by a group selected from -N**R⁹** -, -O-, -S(O)ₚ -, -CO₂ -, -O₂C-, -CON**R⁹** -, -N**R⁹**CO-, -N**R⁹** CON**R¹⁰** -, -CO-, -CH(OH)-, alkenyl or alkynyl and the alkyl may be unsubstituted or substituted with one or more substituents selected from:
      (i) aryl, wherein aryl is as defined above, (ii) substituted aryl wherein aryl is as defined above and in which the substituents are **X'**, **Y'** and **Z'**, (iii) heteroaryl, wherein heteroaryl is as defined above, (iv) substituted heteroaryl wherein heteroaryl is as defined above and in which the substituents are **X'**, **Y'**, and **Z'**, (v) unsubstituted or substituted aryloxy, in which the substituents on aryl are **X'**, **Y'**, and **Z'**, (vi) -O**R⁹**, (vii) -O**R¹¹**, (viii) -OCO**R⁹**, (ix) -OCO₂**R⁹**, (x) -N**R⁹ R¹⁰**, (xi) -CHO, (xii) -N**R⁹** COC1-6 alkyl-**R¹⁰**, (xiii) -N**R⁹** CO₂ C1-6 alkyl-**R¹⁰**, (xiv) -N**R⁹** CON**R⁹ R¹⁰**, (xv) -OCON**R⁹ R¹⁰**, (xvi) -CON**R⁹ R¹⁰**,
   (d) halogen,
   (e) -N**R⁹ R¹⁰**,
   (f) -CN,
   (g) -CHO,
   (h) -CF₃,
   (i) -S**R¹⁵**, wherein **R¹⁵** is hydrogen, C1-6 alkyl, trifluoromethyl, or phenyl,
   (j) -SO**R¹⁵**,
   (k) -SO₂ **R¹⁵**,
   (l) -CON**R⁹ R¹⁰**,
   (m) **R¹⁶** O(CH₂)ₘ - wherein **R¹⁶** is hydrogen, C1-6 alkyl, hydroxy-C2-3 alkyl, -CF₃, phenyl, **R¹¹** or naphthyl and m is 0, 1, 2, or 3,
   (n) -CH(O**R¹⁷**)(O**R¹⁸**), wherein **R¹⁷** and **R¹⁸** are C1-3 alkyl or taken together form an ethyl or propyl bridge,
   (o) 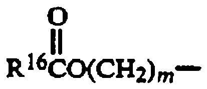 wherein **R¹⁶** and m are as defined above,
   (p) 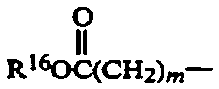 wherein **R¹⁶** and m are as defined above, and
   (q) -**R¹¹** ; or any two of **X**, **Y** and **Z** may be joined to form a saturated ring having 5, 6 or 7 ring atoms, said ring atoms comprising 1 or 2 oxygen atoms, the remaining ring atoms being carbon;

**X'**, **Y'** and **Z'** independently are selected from:
(a) hydrogen,
(b) C1-7 alkyl,
(c) C2-6 alkenyl,
(d) halogen,
(e) -(CH₂)ₘ -N**R⁹ R¹⁰**, wherein **R⁹**, **R¹⁰** and m are as defined above,
(f) -CN,
(g) -CHO,
(h) -CF₃,
(i) -S**R¹⁵**, wherein **R¹⁵** is as defined above,
(j) -SO**R¹⁵**, wherein **R¹⁴** is as defined above,
(k) -SO2**R¹⁵**, wherein **R¹⁵** is as defined above,
(l) -CON**R⁹ R¹⁰**, wherein **R⁹** and **R¹⁰** are as defined above,
(m) **R¹⁶** O(CH₂)ₘ -, wherein **R¹⁶** and m are as defined above,
(n) -CH(O**R¹⁷**)(O**R¹⁸**), wherein **R¹⁷** and **R¹⁸** are as defined above,
(o) 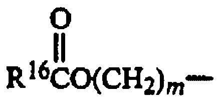 wherein **R¹⁶** and m are as defined above,
(p) 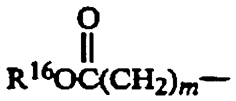 wherein **R¹⁶** and m are as defined above, and
(q) -**R¹¹**.

Compounds of the formula according to claim 1 of the reference US6440990B1 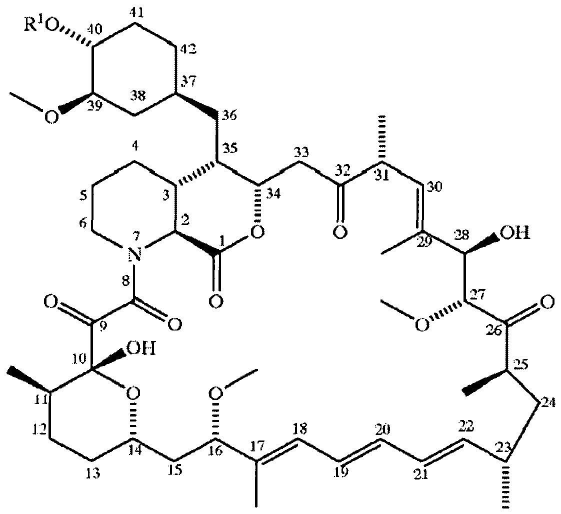 wherein **R¹** is hydroxy(C1-6)alkyl or hydroxy(C1-3)alkoxy(C1-3)alkyl; or 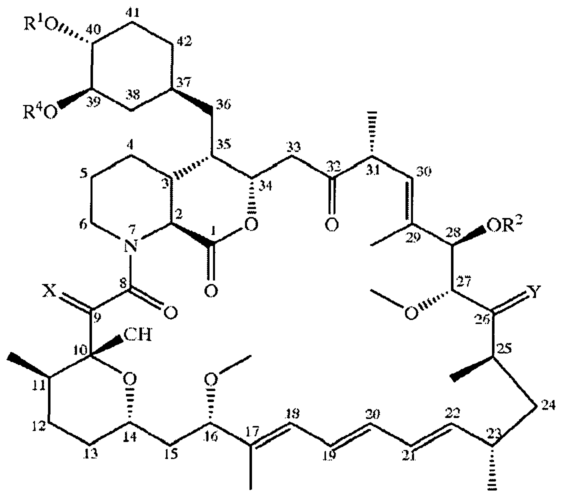 wherein
**X** is (H,H) or O
**Y** is (H,OH) or O;
**R¹** and **R²** are independent selected from:
H, alkyl, thioalkyl, arylalkyl, hydroxyalkyl, dihydroxyalkyl, hydroxyalkylarylalkyl, dihydroxyalkylaryalkyl, alkoxyalkyl, acyloxyalkyl,aminoalkyl, alkylaminoalkyl, alkoxycarbonylaminoalkyl, acylaminoalkyl,arylsulfonamidoalkyl, allyl, dihydroxyalkylallyl, dioxolanylallyl, carbalkoxyalkyl, and (**R³)**₃Si where each **R³** is independenty selected from H, methyl, ethyl, isopropyl, t-butyl, and phenyl; wherin "alkl-"or "alky-" refers to C1-6 alkyl branched or linear preferably C1-3 alkyl, in which the carbon chain may be optionally interrupted by an ether (-0-) linkage; and
**R⁴** is methyl or **R⁴** and **R¹** together form C2-5 alkylene;
provided that **R¹** and **R²** are not both H; and
provided that where **R¹** is (**R³**)₃Si or carbalkoxyalkyl, **X** and **Y** are not both O.

Compounds of the formula according to claim 1 of the reference US7091213B2 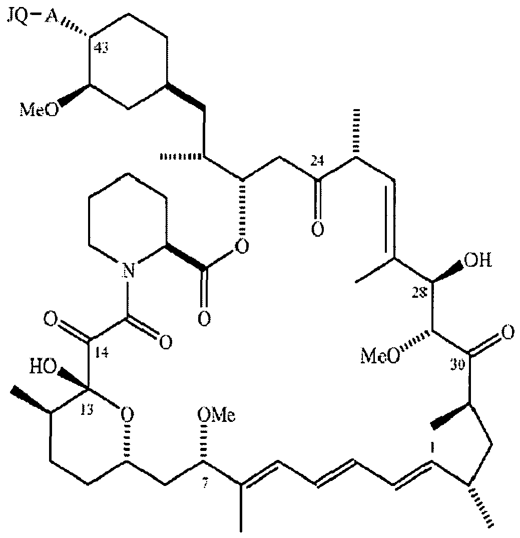 wherein **A** is -O-, -S- or -**NR²** - or is absent;
**Q** is absent or (if **A** is -O-, -S- or -NR²-) **Q** may be -**V**-, -O**V**-, -S**V**-, or -N**R**² **V**-, where **V** is an aliphatic, heteroaliphatic, aryl, or heteroaryl moiety, such that **J** is linked to the cyclohexyl ring directly, through **A** or through **VA**, O**VA**, S**VA** or N**R**² **VA**: 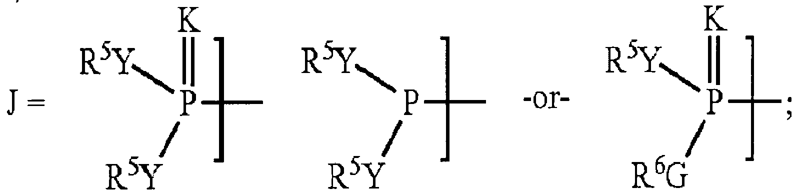 **K** is **O** or S; each occurrence of **Y** is independently -O-, -S-, -N**R**² -, or a bond linking a **R⁵** moiety to P;
each occurrence of **R²** and **R⁵** is independently an aliphatic, heteroaliphatic, aryl, or heteroaryl moiety, or H; and each occurrence of **R⁶** is independently -P**K**(**YR**⁵ )(**YR**⁵ ), -SO₂(**YR**⁵ ) or -C(O)(**YR**⁵ ); so long as any **R**² or **R**⁵ moiety linked directly to P is not H;
wherein two **R²** , **R⁵** and/or **R⁶** moieties may be chemically linked to one another to form a ring;
each occurrence of **G** is independently -O-, -S-, -N**R²** - or (**M**)ₓ; each occurrence of **M** is independently a substituted or unsubstituted methylene moiety, and any **M-M'** moiety may be saturated or unsaturated; each occurrence of x is independently an integer from 1-6; wherein each of the foregoing aliphatic and heteroaliphatic moieties is independently linear or branched, or cyclic or acyclic, and substituted or unsubstituted, and each of the aryl, heteoraryl, acyl, aroyl or heteroaroyl moieties is independently substituted or unsubstituted;
with the proviso that: **J-Q-A-** is not (HO)₂(P-O)O-; (MeO)₂(P-O)O-; (**R² Y**)(Me)(P-O)O-, where (**R² Y**) comprises an immunogenic carrier material, detector carrier material or a solid matrix, or (HO)₂(P-O)-**W**-O- (or a desmethyl or reduced analog of such (HO)₂(P-O)-**W**-O-containing rapamycin derivative, where **W** comprises a substituted or unsubstituted heterocycle comprising alone or fused to a six-membered aromatic ring, 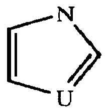 wherein **U** is substituted or unsubstituted amino, O, S, SO or SO₂.

Compounds of the formula according to claim 1 of the reference US7220755B2 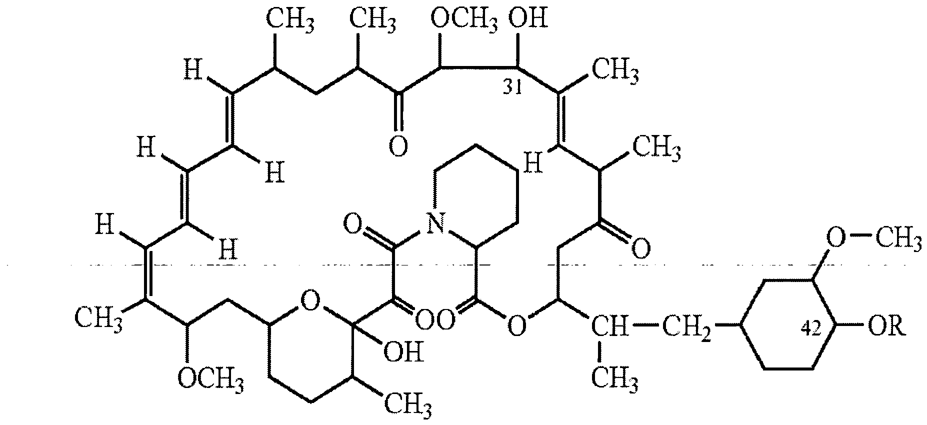 wherein **R** is **R^{a}** -O-**R^{b}** , where Ra is C2-6 alkylene and **R**^{b} is C1-5 alkyl, and where the number of carbon atoms in the sum of **R**^{a} and **R^{b}** is 7 or fewer. In various embodiments, **R** is of the form -(CH₂)ₙ-O-(CH₂)ₘH, where n is from 2 to 6 and m is from 1 to 5; n is 2-5 and m is 1-4; n is 2 and m is 1 or 2; n is 2 and m is 1; and n is 2 and m is 2. In other embodiments, the number of carbon atoms in the sum of **R^{a}** and **R^{b}** is 6 or fewer, preferably 5 or fewer, more preferably **4** or fewer.

Compounds of the formula according to claim 1 of the reference US7648996B2 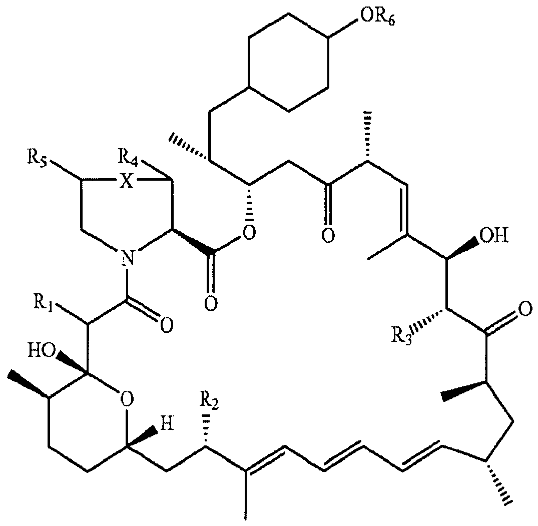 wherein:
**X** represents bond or CH₂;**R**¹ represents a keto group or (H,H); **R²** represents OH or OMe; **R³** represents H, OH or OMe;**R⁴** and **R⁵** each independently represent H or OH;**R⁶** represents -**R⁷**, -C(O)**R⁷**, -(CH₂)₂-O-[C**R**^{**2**1}**R²²**-O]ₐ-C(O)-**R²³**; -C**R**^{**2**0}**R²²**-O-C(O)-**R²³**; -PO**R¹⁹R²⁰**, -PO(O**R¹⁹**) (O**R²⁰**) or Y-**R¹⁵;R⁷** represents - (C**R⁸R⁹**)ₘ(C**R¹⁰R¹¹**)ₚC**R¹²R¹³R¹⁴**; **R⁸** and **R⁹** each independently represent C1-C4 alkyl, C2-C4 alkenyl or C2-C4 alkynyl, any of which groups may optionally be substituted with -PO(OH)₂, -CF₂PO(OH)₂, -OH, -COOH or -NH₂; or R8 and R9 each independently represent H, trifluoromethyl or F; **R¹⁰**, **R¹¹**, **R¹²**, **R¹³** and **R¹⁴** each independently represent C1-C4 alkyl, C2-C4 alkenyl or C2-C4 alkynyl, any of which groups may optionally be substituted with - PO(OH)₂, -CF₂PO(OH)₂, -OH, -COOH or -NH₂; or **R¹⁰, R¹¹, R¹², R¹³** and **R¹⁴** may be independently selected from H, -(C**R⁸R⁹**)_{q}NH₂, -(C**R⁸R⁹**)_{q}OH, CF₃, F, COOH; or **R¹⁰** and **R¹¹** or **R¹²** and **R¹³** or **R¹³** and **R¹⁴** may be taken together with the carbon to which they are joined to form a C3-C6 cycloalkyl or a 3 to 6 membered heteroalkyl ring that contains one or more heteroatoms selected from N, O and S and that is optionally, substituted with up to 5-(C**R⁸R⁹**)_{q}OH, -(C**R⁸R⁹**)qNH₂ or COOH groups; **Y**=bond, -C(O)-O-; -(CH₂)₂-O-C(O)-O-;
**R¹⁵** represents 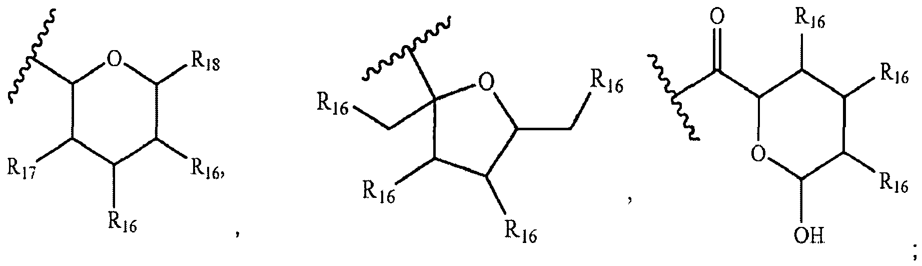
**R¹⁶** are each independently H or OH;**R¹⁷** is independently selected from H, OH and NH₂;**R¹⁸** is independently selected from H, -CH₃, -CH₂OH and - COOH;provided however that no more than 2 groups selected from **R¹⁶**, **R¹⁷** and **R¹⁸** represent H or CH₃;**R¹⁹** and R²⁰ each independently represent H or C1-C4 alkyl or **R¹⁹** and **R²⁰** together represent -CH₂; **R²¹** is independently selected from H, CH₃;**R²²** is independently selected from H, -CH₃, -CH-CH₂, -CH₂Cl, -CHCl₂,-CCl₃, -CH(OH)Me, -CH₂OH, -CH₂CH₃, -CH(Cl)Me; **R²³** is independently **R⁷, Y-R¹⁵** or a 5 or 6 membered aryl or heteroaryl ring optionally substituted with between one and three groups selected from OH, F, Cl, Br, NO₂ and NH₂;a represents 0 or 1;m, p and q each independently represent an integer between 0-4;provided however that the **R⁷** moiety does not contain more than 12 carbon atoms and does contain at least one functional group selected from -PO(OH)₂, - CF₂PO(OH)₂, -COOH, OH or NH₂;

Compounds of the formula according to claim 1 of the reference US7659244B2 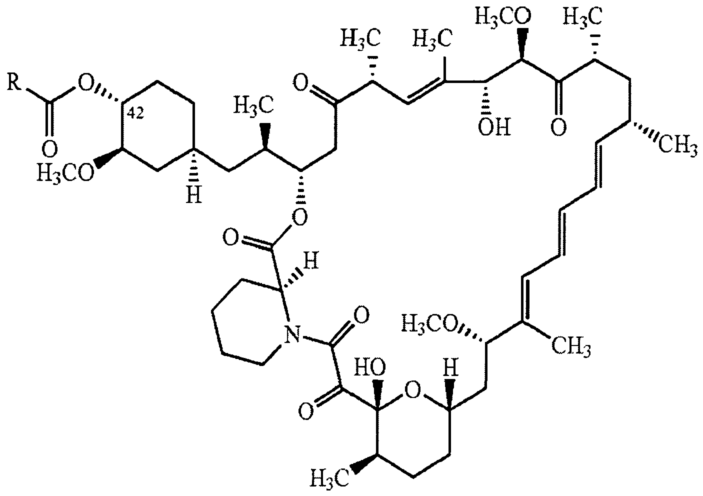 wherein,
**R** is NH-(**A**)ₙ-CH₂OH; 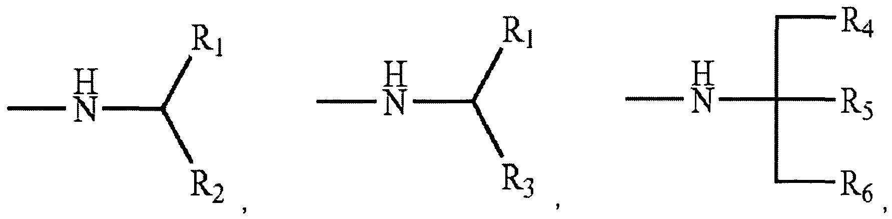 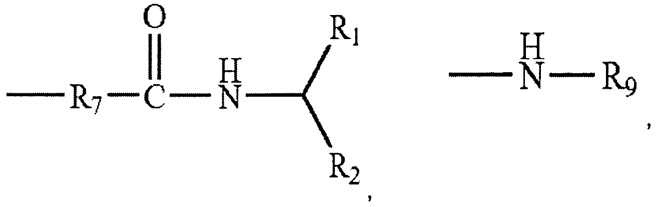 **A** is D or L amino acid and n=1-10,
**R¹** and **R²** are each independently, hydrogen, alkyl of 1-6 carbons atoms, hydroxyalkyl of 1-6 carbon atoms, or CO₂**R⁹**,
**R³** is Ar,
**R⁴**, **R⁵** and **R⁶** are each independently alkyl of 1-6 carbon atoms or hydroxyalkyl of 1-6 carbon atoms,
**R⁷** and **R⁸** are each independently hydrogen, cycloalkyl of 1-6 carbon atoms or hydroxycycloalkyl of 3-16 carbon atoms, and
**R⁹** is alkyl of 1-6 carbon atoms,
**R¹⁰** is alkyl of 1-10 carbon atoms and
**R¹¹** is cycloalkoxyalkyl of 3-10 carbon atoms.
and wherein **R** and said compound of formula are linked through a carbamate ester linkage.

Principally, a FKBP subtype-specific rapamycin analogue can be used for treatment of all those diseases, wherein the inhibition of mTOR activity is beneficial for treatment of said disease. The use of a FKBP subtype-specific rapamycin analogue is especially preferred in those diseases, wherein the localization of the FKBP subtype is restricted to the pathologically changed or disturbed tissues and cells. In a further embodiment the use of a FKBP subtype-specific rapamycin analogue is preferred in diseases, wherein the FKBP subtype is concentrated, more abundant or more active in the pathologically changed or disturbed tissues and cells. The use of a FKBP subtype-specific rapamycin analogue is also preferred in diseases, wherein the expression of the FKBP subtype is not only restricted to said pathologically changed tissues and cells, however, wherein the expression of the FKBP subtype in other tissues and cells does not mediate any undesirable side effects.

Thus, the present findings provide the rationale and a structural basis for the use of a FKBP subtype-specific rapamycin analogue as an inhibitor for tissue and/or cell-type specific mTOR inhibition. As to date it has not been suggested that other FKBP-rapamycin complexes, especially the FKBP51-rapamycin complex, can inhibit mTOR activity. By using a FKBP subtype-specific rapamycin analogue that specifically binds e.g. only FKBP51, inhibition of mTOR can be restricted to those cells and tissues, which express only FKBP51. Potential clinical indications are transplantation, cancer, depression, neurodegeneration, and metabolic disorders.

Consequently, in one embodiment the FKBP subtype-specific rapamycin analogue is selective for a single FKBP, wherein the FKBP subtype-specific rapamycin analogue selective for the single FKBP binds or inhibits mTOR or its FRB domain preferentially in a complex with the single, specific human FKBP. This means that the FKBP subtype-specific rapamycin analogue preferentially is not able to form a mTOR-inhibitory complex with other FKBPs other than the single FKBP.

In another embodiment the FKBP subtype-specific rapamycin analogue is selective for two FKBPs and binds or inhibits mTOR or its FRB domain preferentially in a complex with one of the two FKBPs. This means that the FKBP subtype-specific rapamycin analogue preferentially is not able to form a mTOR-inhibitory complex with other FKBPs other than the two FKBPs.

In another embodiment the FKBP subtype-specific rapamycin analogue is selective for three FKBPs and binds or inhibits mTOR or its FRB domain preferentially in a complex with one of the three FKBPs. This means that the FKBP subtype-specific rapamycin analogue preferentially is not able to form a mTOR-inhibitory complex with other FKBPs other than the three FKBPs.

In yet another embodiment the FKBP subtype-specific rapamycin analogue is selective for four FKBPs and binds or inhibits mTOR or its FRB domain preferentially in a complex with one of the four FKBPs. This means that the FKBP subtype-specific rapamycin analogue preferentially is not able to form a mTOR-inhibitory complex with other FKBPs other than the four FKBPs.

The expression "mTOR-inhibitory complex" as used herein refers to a complex comprising the FKBP and the FKBP subtype-specific rapamycin analogue.

Not being able to form a mTOR-inhibitory complex with other FKBPs preferably means that the FKBP subtype-specific rapamycin analogue is tested for its ability to (i) bind FRB/mTOR or (ii) inhibition of mTOR in the context of preferentially one FKBP homolog.
(i) FRB/mTOR binding can be determined in a FRET-based protein-protein dimerization assay according to assay example 2, wherein preferred compounds have an EC₅₀ value in this assay of < 1µM, preferably < 500nM, preferably < 200nM, even more preferably < 100nM, more preferably < 20nM in the presence of the preferred FKBP and the EC₅₀ in this assay should be preferably at least 5-fold lower, further preferred at least 10-fold lower, preferably 15-fold lower, more preferred 20-fold lower, even more preferred 25-fold lower, further preferred 30-fold lower, preferably 35-fold lower, more preferred 50-fold lower, even more preferred 100-fold lower and most preferred 500-fold lower for the preferred FKBP than for at least two other FKBPs, preferably four other FKBPs, more preferably all other cytosolic FKBPs.

In a preferred embodiment the FKBP subtype-specific rapamycin analogue has an EC₅₀ value of < 1 µM for its preferred FKBP and the EC₅₀ value is 5-fold lower than for at least two other FKBPs. In another embodiment the FKBP subtype-specific rapamycin analogue has an EC₅₀ value of < 500nM for its preferred FKBP and the EC₅₀ value is 10-fold lower for at least two other FKBPs. In a further preferred embodiment the FKBP subtype-specific rapamycin analogue has an EC₅₀ value of < 200nM for its preferred FKBP and the EC₅₀ value is 20-fold lower for at least two other FKBPs. In yet another embodiment the FKBP subtype-specific rapamycin analogue has an EC₅₀ value of <100nM for its preferred FKBP and the EC₅₀ value is 25-fold lower for at least two other FKBPs. In another embodiment the FKBP subtype-specific rapamycin analogue has an EC₅₀ value of <20nM for its preferred FKBP and the EC₅₀ value is 50-fold lower for at least two other FKBPs. In another embodiment the FKBP subtype-specific rapamycin analogue has an EC₅₀ value of < 500nM for its preferred FKBP and the EC₅₀ value is 5-fold lower for at least two other FKBPs. In a further preferred embodiment the FKBP subtype-specific rapamycin analogue has an EC₅₀ value of < 200nM for its preferred FKBP and the EC₅₀ value is 10-fold lower for at least two other FKBPs. In yet another embodiment the FKBP subtype-specific rapamycin analogue has an EC₅₀ value of <100nM for its preferred FKBP and the EC₅₀ value is 20-fold lower for at least two other FKBPs. In another embodiment the FKBP subtype-specific rapamycin analogue has an EC₅₀ value of <20nM for its preferred FKBP and the EC₅₀ value is 25-fold lower for at least two other FKBPs.
(ii) FKBP subtype-dependent mTOR inhibition can be experimentally defined by assay example 1. Preferred compounds should have an IC₅₀ in this assay of preferably < 1µM, preferably < 500nM, preferably < 200nM, even more preferably < 100nM, more preferably < 20nM and most preferred < 2nM in the presence of the preferred FKBP and the IC₅₀ in this assay should be preferably at least 5-fold lower, further preferred at least 10-fold lower, preferably 15-fold lower, more preferred 20-fold lower, even more preferred 25-fold lower, further preferred 30-fold lower, preferably 35-fold lower, more preferred 50-fold lower, even more preferred 100-fold lower and most preferred 500-fold lower for the preferred FKBP than for at least two other FKBPs, preferably four other FKBPs, more preferably all other cytosolic FKBPs.

In a preferred embodiment the FKBP subtype-specific rapamycin analogue has an IC₅₀ value of < 1µM for its preferred FKBP and the EC₅₀ value is 5-fold lower than for at least two other FKBPs. In another embodiment the FKBP subtype-specific rapamycin analogue has an IC₅₀ value of < 500nM for its preferred FKBP and the IC₅₀ value is 10-fold lower for at least two other FKBPs. In a further preferred embodiment the FKBP subtype-specific rapamycin analogue has an IC₅₀ value of < 200nM for its preferred FKBP and the IC₅₀ value is 20-fold lower for at least two other FKBPs. In yet another embodiment the FKBP subtype-specific rapamycin analogue has an IC₅₀ value of <100nM for its preferred FKBP and the IC₅₀ value is 25-fold lower for at least two other FKBPs. In another embodiment the FKBP subtype-specific rapamycin analogue has an IC₅₀ value of <20nM for its preferred FKBP and the IC₅₀ value is 50-fold lower for at least two other FKBPs. In another embodiment the FKBP subtype-specific rapamycin analogue has an IC₅₀ value of < 500nM for its preferred FKBP and the IC₅₀ value is 5-fold lower for at least two other FKBPs. In a further preferred embodiment the FKBP subtype-specific rapamycin analogue has an IC₅₀ value of < 200nM for its preferred FKBP and the IC₅₀ value is 10-fold lower for at least two other FKBPs. In yet another embodiment the FKBP subtype-specific rapamycin analogue has an IC₅₀ value of <100nM for its preferred FKBP and the IC₅₀ value is 20-fold lower for at least two other FKBPs. In another embodiment the FKBP subtype-specific rapamycin analogue has an IC₅₀ value of <20nM for its preferred FKBP and the IC₅₀ value is 25-fold lower for at least two other FKBPs.

"Preferred FKBP", as used herein, refers to the specific FKBP the subtype-specific rapamycin analogue is selective for. In those embodiments, wherein the FKBP subtype-specific rapamycin analogue is selective for more than one FKBP, the "preferred FKBP" is the FKBP with the lowest EC₅₀ value selected from the FKBPs the rapamycin analogue is selective for. Hence, if e.g. the FKBP subtype-specific rapamycin analogue is selective for FKBP12 and FKBP51, the "preferred FKBP" would be the one with the lower EC₅₀ value.

The FKBP subtype-specific rapamycin analogue can be used as an active ingredient, together with at least one pharmaceutically acceptable carrier, excipient and/or diluents for use in a pharmaceutical composition.

Such pharmaceutical compositions comprise the FKBP subtype-specific rapamycin analogue as an active ingredient, together with at least one pharmaceutically acceptable carrier, excipient, binders, disintegrates, glidents, diluents, lubricants, coloring agents, sweetening agents, flavoring agents, preservatives or the like. The pharmaceutical composition can be prepared in a conventional solid or liquid carrier or diluents and a conventional pharmaceutically-made adjuvant at suitable dosage level in a known way.

Preferably the FKBP subtype-specific rapamycin analogue is suitable for intravenous administration or suitable for oral administration or suitable for administration by inhalation.

Administration forms include, for example, pills, tablets, film tablets, coated tablets, capsules, liposomal formulations, micro- and nano-formulations, powders and deposits. Furthermore, the pharmaceutical preparations are suitable for parenteral application, including dermal, intradermal, intragastral, intracutan, intravasal, intravenous, intramuscular, intraperitoneal, intranasal, intravaginal, intrabuccal, percutan, rectal, subcutaneous, sublingual, topical, or transdermal application, which preparations in addition to typical vehicles and/or diluents contain the FKBP subtype-specific rapamycin analogue.

The FKBP subtype-specific rapamycin analogue can also be administered in form of its pharmaceutically active salts. Suitable pharmaceutically active salts comprise acid addition salts and alkali or earth alkali salts. For instance, sodium, potassium, lithium, magnesium or calcium salts can be obtained.

The FKBP subtype-specific rapamycin analogue forms pharmaceutically acceptable salts with organic and inorganic acids. Examples of suitable acids for such acid addition salt formation are hydrochloric acid, hydrobromic acid, sulfuric acid, phosphoric acid, acetic acid, citric acid, oxalic acid, malonic acid, salicylic acid, p-aminosalicylic acid, malic acid, fumaric acid, succinic acid, ascorbic acid, maleic acid, sulfonic acid, phosphonic acid, perchloric acid, nitric acid, formic acid, propionic acid, gluconic acid, lactic acid, tartaric acid, hydroxymaleic acid, pyruvic acid, phenylacetic acid, benzoic acid, p-aminobenzoic acid, p-hydroxybenzoic acid, methanesulfonic acid, ethanesulfonic acid, nitrous acid, hydroxyethanesulfonic acid, ethylenesulfonic acid, p-toluenesulfonic acid, naphthylsulfonic acid, sulfanilic acid, camphersulfonic acid, china acid, mandelic acid, o-methylmandelic acid, hydrogen-benzenesulfonic acid, picric acid, adipic acid, Do-tolyltartaric acid, tartronic acid, α-toluic acid, (o, m, p)-toluic acid, naphthylamine sulfonic acid, and other mineral or carboxylic acids well known to those skilled in the art. The salts are prepared by contacting the free base form with a sufficient amount of the desired acid to produce a salt in the conventional manner.

The pharmaceutical compositions will typically be administered together with suitable carrier materials selected with respect to the intended form of administration, i.e. for oral administration in the form of tablets, capsules (either solid filled, semi-solid filled or liquid filled), powders for constitution, aerosol preparations consistent with conventional pharmaceutical practices. Other suitable formulations are gels, elixirs, dispersible granules, syrups, suspensions, creams, lotions, solutions, emulsions, suspensions, dispersions, and the like. Suitable dosage forms for sustained release include tablets having layers of varying disintegration rates or controlled release polymeric matrices impregnated with the active components and shaped in tablet form or capsules containing such impregnated or encapsulated porous polymeric matrices. The pharmaceutical compositions may be comprised of 5 to 95% of the FKBP subtype-specific rapamycin analogue.

As pharmaceutically acceptable carrier, excipient and/or diluents can be used lactose, starch, sucrose, cellulose, magnesium stearate, dicalcium phosphate, calcium sulfate, talc, mannitol, ethyl alcohol (liquid filled capsules).

Suitable binders include starch, gelatin, natural sugars, corn sweeteners, natural and synthetic gums such as acacia, sodium alginate, carboxymethyl-cellulose, polyethylene glycol and waxes. Among the lubricants that may be mentioned for use in these dosage forms, boric acid, sodium benzoate, sodium acetate, sodium chloride, and the like. Disintegrants include starch, methylcellulose, guar gum and the like. Sweetening and flavoring agents and preservatives may also be included where appropriate. Some of the terms noted above, namely disintegrants, diluents, lubricants, binders and the like, are discussed in more detail below.

Additionally, the compositions may be formulated in sustained release form to provide the rate controlled release of any one or more of the components or active ingredients to optimize the therapeutic effects. Suitable dosage forms for sustained release include layered tablets containing layers of varying disintegration rates or controlled release polymeric matrices impregnated with the active components and shaped in tablet form or capsules containing such impregnated or encapsulated porous polymeric matrices. Liquid form preparations include solutions, suspensions and emulsions. As an example may be mentioned water or water-propylene glycol solutions for parenteral injections or addition of sweeteners and opacifiers for oral solutions, suspensions and emulsions. Liquid form preparations may also include solutions for intranasal administration. Aerosol preparations suitable for inhalation may include solutions and solids in powder form, which may be in combination with a pharmaceutically acceptable carrier such as inert compressed gas, e.g. nitrogen.

Also possible are solid form preparations which are intended to be converted, shortly before use, to liquid form preparations for either oral or parenteral administration. Such liquid forms include solutions, suspensions and emulsions.

The term capsule refers to a special container or enclosure made of methyl cellulose, polyvinyl alcohols, or denatured gelatins or starch for holding or containing compositions comprising the active ingredients. Hard shell capsules are typically made of blends of relatively high gel strength bone and pork skin gelatins. The capsule itself may contain small amounts of dyes, opaquing agents, plasticizers and preservatives.

Tablet means compressed or molded solid dosage form containing the active ingredients with suitable diluents. The tablet can be prepared by compression of mixtures or granulations obtained by wet granulation, dry granulation or by compaction well known to a person skilled in the art.

Powders for constitution refer to powder blends containing the active ingredients and suitable diluents which can be suspended in water or juices.

Suitable diluents are substances that usually make up the major portion of the composition or dosage form. Suitable diluents include sugars such as lactose, sucrose, mannitol and sorbitol, starches derived from wheat, corn rice and potato, and celluloses such as microcrystalline cellulose. The amount of diluents in the composition can range from about 5 to about 95% by weight of the total composition, preferably from about 25 to about 75%, more preferably from about 30 to about 60% by weight, and most preferably from about 40 to 50% by weight.

The term disintegrants refers to materials added to the composition to help it break apart (disintegrate) and release the medicaments. Suitable disintegrants include starches, "cold water soluble" modified starches such as sodium carboxymethyl starch, natural and synthetic gums such as locust bean, karaya, guar, tragacanth and agar, cellulose derivatives such as methylcellulose and sodium carboxymethylcellulose, microcrystalline celluloses and cross-linked microcrystalline celluloses such as sodium croscarmellose, alginates such as alginic acid and sodium alginate, clays such as bentonites, and effervescent mixtures. The amount of disintegrant in the composition can range from about 1 to about 40% by weight of the composition, preferably 2 to about 30% by weight of the composition, more preferably from about 3 to 20% by weight of the composition, and most preferably from about 5 to about 10% by weight.

Binders characterize substances that bind or "glue" powders together and make them cohesive by forming granules, thus serving as the "adhesive" in the formulation. Binders add cohesive strength already available in the diluents or bulking agent. Suitable binders include sugars such as sucrose, starches derived from wheat, corn rice and potato; natural gums such as acacia, gelatin and tragacanth; derivatives of seaweed such as alginic acid, sodium alginate and ammonium calcium alginate; cellulosic materials such as methylcellulose and sodium carboxymethylcellulose and hydroxypropyl-methylcellulose; polyvinylpyrrolidone; and inorganics such as magnesium aluminum silicate. The amount of binder in the composition can range from about 1 to 30% by weight of the composition, preferably from about 2 to about 20% by weight of the composition, more preferably from about 3 to about 10% by weight, even more preferably from about 3 to about 6% by weight.

Lubricant refers to a substance added to the dosage form to enable the tablet, granules, etc. after it has been compressed, to release from the mold or die by reducing friction or wear. Suitable lubricants include metallic stearates such as magnesium stearate, calcium stearate or potassium stearate; stearic acid; high melting point waxes; and water soluble lubricants such as sodium chloride, sodium benzoate, sodium acetate, sodium oleate, polyethylene glycols and d'I-leucine. Lubricants are usually added at the very last step before compression, since they must be present on the surfaces of the granules and in between them and the parts of the tablet press. The amount of lubricant in the composition can range from about 0.05 to about 15% by weight of the composition, preferably 0.2 to about 5% by weight of the composition, more preferably from about 0.3 to about 3%, and most preferably from about 0.3 to about 1.5% by weight of the composition.

Glidents are materials that prevent caking and improve the flow characteristics of granulations, so that flow is smooth and uniform. Suitable glidents include silicon dioxide and talc. The amount of glident in the composition can range from about 0.01 to 10% by weight of the composition, preferably 0.1% to about 7% by weight of the total composition, more preferably from about 0.2 to 5% by weight, and most preferably from about 0.5 to about 2% by weight.

Coloring agents are excipients that provide coloration to the composition or the dosage form. Such excipients can include food grade dyes and food grade dyes adsorbed onto a suitable adsorbent such as clay or aluminum oxide. The amount of the coloring agent can vary from about 0.01 to 10% by weight of the composition, preferably from about 0.05 to 6% by weight, more preferably from about 0.1 to about 4% by weight of the composition, and most preferably from about 0.1 to about 1%.

The term buffer, buffer system, buffer solution and buffered solution, when used with reference to hydrogen-ion concentration or pH, refers to the ability of a system, particularly an aqueous solution, to resist a change of pH on adding acid or alkali, or on dilution with a solvent. Preferred buffer systems can be selected from the group consisting of formate (pKa=3.75), lactate (pKa=3.86), benzoic acid (pKa=4.2) oxalate (pKa=4.29), fumarate (pKa=4.38), aniline (pKa=4.63), acetate buffer (pKa=4.76), citrate buffer (pKa2=4.76,pKa3=6.4), glutamate buffer (pKa=4.3), phosphate buffer (pKa=7.20), succinate (pKa1=4.93;pKa2=5.62), pyridine (pKa=5.23), phthalate (pKa=5.41); histidine (pKa=6.04), MES (2-(N-morpholino)ethanesulphonic acid; pKa=6.15); maleic acid (pKa=6.26); cacodylate (dimethylarsinate, pKa=6.27), carbonic acid (pKa=6.35), ADA (N-(2-acetamido)imino-diacetic acid (pKa=6.62); PIPES (4-piperazinebis-(ethanesulfonic acid; BIS-TRIS-propane (1,3-bis[tris(hydroxymethyl)methylamino]-propane), pKa=6.80), ethylendiamine (pKa=6.85), ACES 2-[(2-amino-2-oxoethyl)amino]ethanesulphonic acid; pKa=6.9), imidazole (pKa=6.95), MOPS (3-(N-morphin)-propansulfonic acid; pKa=7.20), diethylmalonic acid (pKa=7.2), TES (2-[tris (hydroxymethyl) methyl] amino ethanesulphonic acid; pKa=7.50) and HEPES (N-2-hydroxylethylpiperazin-N'-2-ethansulfonic acid; pKa=7.55) buffers or other buffers having a pKa between 3.8 to 7.7. Preferred is the group of carboxylic acid buffers such as acetate and carboxylic diacid buffers such as fumarate, tartrate and phthalate and carboxylic triacid buffers such as citrate. Another group of preferred buffers is represented by inorganic buffers such as sulfate, borate, carbonate, oxalate, calcium hydroxyde and phosphate buffers. Another group of preferred buffers are nitrogen containing buffers such as imidazole, diethylenediamine, and piperazine.

Also preferred are sulfonic acid buffers such as TES, HEPES, ACES, PIPES, [(2-hydroxy-1,1-bis(hydroxymethyl)ethyl)amino]-1-propanesulfonic acid (TAPS), 4-(2-hydroxyethyl)piperazine-1-propanesulfonic acid (EPPS), 4-Morpholinepropanesulfonic acid (MOPS) and N,N-bis(2-hydroxyethyl)-2-aminoethanesulfonic acid (BES).

Another group of preferred buffers are glycine buffers such as glycine, glycyl-glycine, glycyl-glycyl-glycine, N,N-bis(2-hydroxyethyl)glycine and N-[2-hydroxy-1,1-bis(hydroxymethyl)ethyl]glycine (Tricine).

Preferred are also amino acid buffers such as glycine, alanine, valine, leucine, isoleucine, serine, threonine, phenylalanine, tyrosine, tryptophane, lysine, arginine, histidine, aspartate, glutamate, asparagine, glutamine, cysteine, methionine, proline, 4-hydroxyproline, N,N,N-trimethyllysine, 3-methylhistidine, 5-hydroxylysine, O-phosphoserine, γ-carboxyglutamate, ε-N-acetyllysine, ω-N-methylarginine, citrulline, ornithine and derivatives thereof.

Also preferred are the following buffers:

| **effective pH range** | **pKa 25°C** | **buffer** |
|---|---|---|
| 2.7-4.2 | 3.40 | malate (pK1) |
| 3.0-4.5 | 3.75 | formate |
| 3.0-6.2 | 4.76 | citrate (pK2) |
| 3.2-5.2 | 4.21 | succinate (pK1) |
| 3.6-5.6 | 4.76 | acetate |
| 3.8-5.6 | 4.87 | propionate |
| 4.0-6.0 | 5.13 | malate (pK2) |
| 4.9-5.9 | 5.23 | pyridine |
| 5.0-6.0 | 5.33 | piperazine (pK1) |
| 5.0-7.4 | 6.27 | cacodylate |
| 5.5-6.5 | 5.64 | succinate (pK2) |
| 5.5-6.7 | 6.10 | MES |
| 5.5-7.2 | 6.40 | citrate (pK3) |
| 5.5-7.2 | 6.24 | maleate (pK2) |
| 5.5-7.4 | 1.70, 6.04, 9.09 | histidine |
| 5.8-7.2 | 6.46 | bis-tris |
| 5.8-8.0 | 7.20 | phosphate (pK2) |
| 6.0-12.0 | 9.50 | ethanolamine |
| 6.0-7.2 | 6.59 | ADA |
| 6.0-8.0 | 6.35 | carbonate (pK1) |
| 6.1-7.5 | 6.78 | ACES |
| 6.1-7.5 | 6.76 | PIPES |
| 6.2-7.6 | 6.87 | MOPSO |
| 6.2-7.8 | 6.95 | imidazole |
| 6.3-9.5 | 6.80,9.00 | BIS-TRIS propane |
| 6.4-7.8 | 7.09 | BES |
| 6.5-7.9 | 7.14 | MOPS |
| 6.8-8.2 | 7.48 | HEPES |
| 6.8-8.2 | 7.40 | TES |
| 6.9-8.3 | 7.60 | MOBS |
| 7.0-8.2 | 7.52 | DIPSO |
| 7.0-8.2 | 7.61 | TAPSO |
| 7.0-8.3 | 7.76 | triethanolamine (TEA) |
| 7.0-9.0 | 0.91, 2.10, 6.70, 9.32 | pyrophosphate |
| 7.1-8.5 | 7.85 | HEPPSO |
| 7.2-8.5 | 7.78 | POPSO |

Preferred are the buffers having an effective pH range of from 2.7 to 8.5, and more preferred of from 3.8 to 7.7. The effective pH range for each buffer can be defined as pKa - 1 to pKa + 1, where Ka is the ionization constant for the weak acid in the buffer and pKa = - log K.

Most preferred are buffers suitable for pharmaceutical use e.g. buffers suitable for administration to a patient such as acetate, carbonate, citrate, fumarate, glutamate, lactate, phosphate, phthalate, and succinate buffers. Particularly preferred examples of commonly used pharmaceutical buffers are acetate buffer, citrate buffer, glutamate buffer and phosphate buffer. Also most preferred is the group of carboxylic acid buffers. The term "carboxylic acid buffers" as used herein shall refer to carboxylic mono acid buffers and carboxylic diacid buffers as well as carboxylic triacid buffers. Of course also combinations of buffers, especially of the buffers mentioned herein are useful.

Some suitable pharmaceutical buffers are a citrate buffer (preferably at a final formulation concentration of from about 20 to 200 mM, more preferably at a final concentration of from about 30 to 120 mM) or an acetate buffer (preferably at a final formulation concentration of about 20 to 200 mM) or a phosphate buffer (preferably at a final formulation concentration of about 20 to 200 mM).

Techniques for the formulation and administration of the peptide may be found in "Remington's Pharmaceutical Sciences" Mack Publishing Co., Easton PA. A suitable composition comprising the peptide mentioned herein may be a solution of the peptide in a suitable liquid pharmaceutical carrier or any other formulation such as tablets, pills, film tablets, coated tablets, dragees, capsules, powders and deposits, gels, syrups, slurries, suspensions, emulsions, and the like.

Further examples of suitable cryo- / lyoprotectants (otherwise referred to as bulking agents or stabilizers) include thiol-free albumin, immunoglobulins, polyalkyleneoxides (e.g. PEG, polypropylene glycols), trehalose, glucose, sucrose, sorbitol, dextran, maltose, raffinose, stachyose and other saccharides (cf. for instance WO 97/29782), while mannitol is used preferably. These can be used in conventional amounts in conventional lyophilization techniques. Methods of lyophilisation are well known in the art of preparing pharmaceutical formulations.

For administration by inhalation the particle diameter of the lyophilised preparation is preferably between 2 to 5 µm, more preferably between 3 to 4 µm. The lyophilised preparation is particularly suitable for administration using an inhalator, for example the OPTINEB^{®} or VENTA-NEB^{®} inhalator (NEBU-TEC, Elsenfeld, Germany). The lyophilised product can be rehydrated in sterile distilled water or any other suitable liquid for inhalation adminstration.

Alternatively for intravenous administration the lyophilised product can be rehydrated in sterile distilled water or any other suitable liquid for intravenous administration.

After rehydration for administration in sterile distilled water or another suitable liquid the lyophilised preparation should have the approximate physiological osmolality of the target tissue for the rehydrated peptide preparation i.e. blood for intravenous administration or lung tissue for inhalation administration. Thus it is preferred that the rehydrated formulation is substantially isotonic.

### Treatment

The term treatment in the context of the present invention refers to the use of the FKBP subtype-specific rapamycin analogue to inhibit, or arrest the symptoms of a disease, wherein the inhibition of mTOR is beneficial for treatment of said disease.

The term "inhibition of mTOR is beneficial" refers to any disease, wherein the abnormal activity or expression of mTOR is causative for the development of said disease. Furthermore, this applies also to diseases, wherein the abnormal activity or expression of mTOR promotes the formation of said disease, without being the primary factor for said disease. Thus, inhibition of mTOR can be beneficial in all diseases that occur wherein the activity or expression of mTOR is abnormal, even though said diseases might also occur in subjects without an abnormal activity or expression of mTOR, however, according to this invention said diseases occur with a greater probability or have a more severe progression in subjects with abnormal mTOR activity or expression. Consequently, treatment with the FKBP subtype-specific rapamycin analogue is especially useful in diseases, wherein the abnormal expression or activity of mTOR aggrevates/promotes the development/occurence of said disease.

The terms "subject" or "patient" are used herein mean any mammal, including but not limited to human beings, including a human patient or subject to which the composition can be administered. The term mammals include human patients and non-human primates, as well as experimental animals such as rabbits, rats, and mice, and other animals.

### Description of the figures

**Figure 1****: Six different FKBP's reduce mTOR activity with nanomolar IC₅₀ values** FRET-based mTOR activity assay, containing purified truncated mTOR and eGFP-4E-BP1 as a substrate: half-logarithmic plot of a rapamycin titration (0 - 1000 nM) against observed FRET ratio (Em 520 nm / Em 495 nm) in presence of 100 nM FKBP protein.
   (•FKBP12; ○FKBP12.6; ▼FKBP13; ΔFKBP25; ■FKBP51; □FKBP52).
**Figure 2****: FKBP's bind to FRB domain of mTOR with high affinity in a rapamycin-dependent manner**
   FRET binding assay with recombinant GST-FRB and eGFP-FKBP's. Half-logarithmic plot of the rapamycin concentration against the observed FRET ratio (Em 520 nm/ Em 495 nm).
   (●FKBP12; ○FKBP12.6; ▼FKBP13; ΔFKBP25; ■FKBP51; □FKBP52).
**Figure 3****: TOR-FKBP51 interactions in eukaryotic cells**
   (A) Overexpression of FKBP51 FK1 restores rapamycin sensitivity in a FKBP-deficient yeast strain: Yeast cells (FKBP deletion strain Δ) were grown for 3 days with or without the expression of human FKBP12 or human FKBP51 FK1 in the absence or presence of 5 µM rapamycin.
   (B) mTOR interacts with FKBP12, FKBP51 and FKBP52 in a rapamycin-dependent manner: HEK-293 cells were transfected with a FLAG-mTOR or FLAG-mTOR-S2035T expression plasmid. 30 minutes prior to lysis, rapamycin, FK506 (final concentration: each 25 nM) or vehicle DMSO were added. The cell lysates were subjected to FLAG immunoprecipitation and the eluates were analysed by immunoblotting.

### Examples

### Example 1: In vitro kinase assay of mTOR activity:

An *in vitro* kinase assay was performed. This assay determines the activity of a heterologously expressed, truncated mTOR protein by assessing phosphorylation of the mTOR substrate 4EBP1. The FRET-based mTOR activity assay (LanthaScreen Kinase assay, Invitrogen) was performed as recommended by the manufacturer. Briefly, a rapamycin dilution series in DMSO was prepared (starting at 400 µM), and an intermediate dilution in kinase reaction buffer was used in the assay. One 386-well contained rapamycin (0 -1 µM), 10 µM ATP, 400 nM eGFP-4EBP1, 625 pg mTOR kinase and 100 nM FKBP in kinase reaction buffer. Mixtures were incubated for 1 h at room temperature before reaction was stopped by addition of an equal amount of EDTA/antibody mixture (final concentrations: 2 nM Tb-labeled anti-GST antibody, 10 mM EDTA in TR-FRET dilution buffer). After 30 minutes of incubation at room temperature, FRET intensity was measured using a Tecan "GENios Pro" reader (FRET 1: Excitation: 340 nm; Emission: 495 nm; FRET 2: Excitation : 340 nm; Emission: 520 nm; Integration : 100 µs lag time ; 200 µs integration time; Number of flashes: 10). The two emission intensities were plotted as ratio (520nm/495nm) against the rapamycin concentration.

Kinase assay with mTORC1 were performed as described by Sato, T., A. Umetsu, and F. Tamanoi (Characterization of the Rheb-mTOR signaling pathway in mammalian cells: constitutive active mutants of Rheb and mTOR. Methods Enzymol., 2008. 438: p. 307-20). Briefly, myc-Raptor was transiently expressed in HEK-293 cells. Transfected cells were starved overnight in DMEM, followed by 40 minutes in PBS, and stimulated with DMEM/10% FBS for 60 minutes. Then, cells were washed once with PBS and resuspended in lysis buffer (40 mM Hepes, pH 7.5; 120 mM NaCl; 1 mM EDTA; 10 mM pyrophosphate; 10 mM glycerophosphate; 0.5 mM orthovanadate; 50 mM NaF; 0.3% (w/v) CHAPS / 1% (v/v) Triton-100; 1% (v/v) protease inhibitor mix), shaken for 20 minutes at 4°C and spun down at full speed for 20 minutes. Myc-affinity beads were added to the lysate and the IP mixture was incubated for 2 h under agitation. Beads were collected and washed five times with lysis buffer. Beads were extracted with mycpeptide in TBS. IP eluate was directly added to recombinant EGFP-4EBP1 or immunoprecipitated inactive HA-S6K in kinase buffer (20 mM Tris/HCl, pH 7.4; 10 mM MgCl2; 0.2 mM ATP). After 30 minutes of incubation at 37°C, the reaction was stopped by addition of SDS sample buffer and boiling for 5 minutes. Finally, the samples were analysed by SDS-PAGE and immunoblotting.

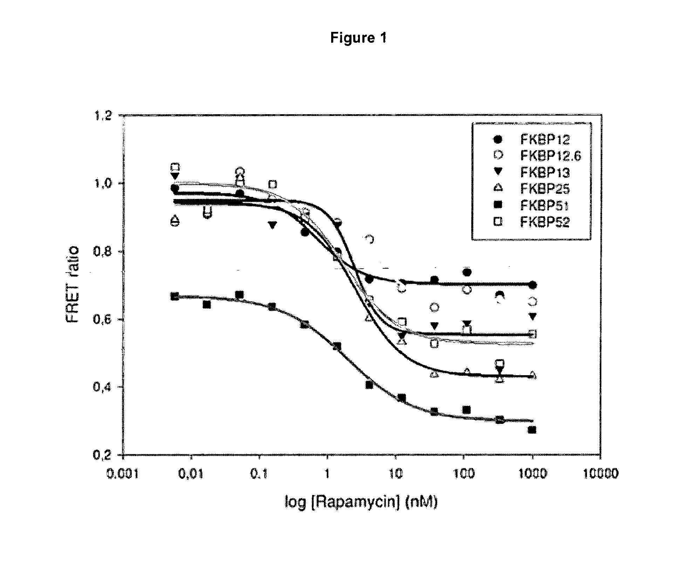

Analysis of the measurement data shows that all examined FKBP proteins (FKBP12, FKBP12.6, FKBP13, FKBP25, FKBP51, FKBP52) exert a potent inhibitory effect on mTOR kinase activity in presence of low nanomolar rapamycin concentrations (Tab. 1 and Fig. 1). For FKBP12, FKBP51 and FKBP52 IC₅₀ values below 2 nM were measured, well below the clinically achieved concentrations of rapamycin.

### Example 2: FRET binding assay with recombinant GST-FRB and eGFP-FKBP's:

A FRET-based protein-protein dimerization assay was used. 5 nM GST-FRB, 100 nM eGFP-FKBP's and 2.5 nM of a Terbium-labelled anti-GST antibody were used. Rapamycin was titrated from 0 to 5 µM.

The time-resolved FRET assay that was used to investigate the interaction between FKBPs and FRB was originally developed by Invitrogen and is based on a Terbium-labelled anti-GST antibody and an eGFP (enhanced green fluorescent protein) acceptor. As GST fusion protein, GST-FRB was used while all FKBPs were utilized as N-terminal eGFP-fusions in the assay. The assay was performed following the manufacturer's recommendations: in a total volume of 10 µl, 5 µl of rapamycin dilution (1:1 dilution in TR-FRET dilution buffer, starting at 4 µM) were combined with 5 µl of protein mixture in low-binding 384-well plates. The protein mixture contained variable amounts of the following components in dilution buffer: 5 nM GST-FRB, 100 nM eGFP-FKBP, 2.5 nM Tb-labelled anti-GST antibody. After 15 minutes of incubation, the wells were read using a Tecan "GENios Pro" reader (FRET 1: Excitation: 340 nm; Emission: 495 nm; FRET 2: Excitation : 340 nm; Emission: 520 nm; Integration : 100 µs lag time ; 200 µs integration time; Number of flashes: 10). The two emission intensities were plotted as ratio (520nm/495nm) against the rapamycin concentration. Then, an EC₅₀ value was calculated by using the "Four-Parameter-Logistic-Curve"-Fitting in SigmaPlot. This value corresponds to the rapamycin concentration at which the half-maximum concentration of the FRB*FKBP complex is present.

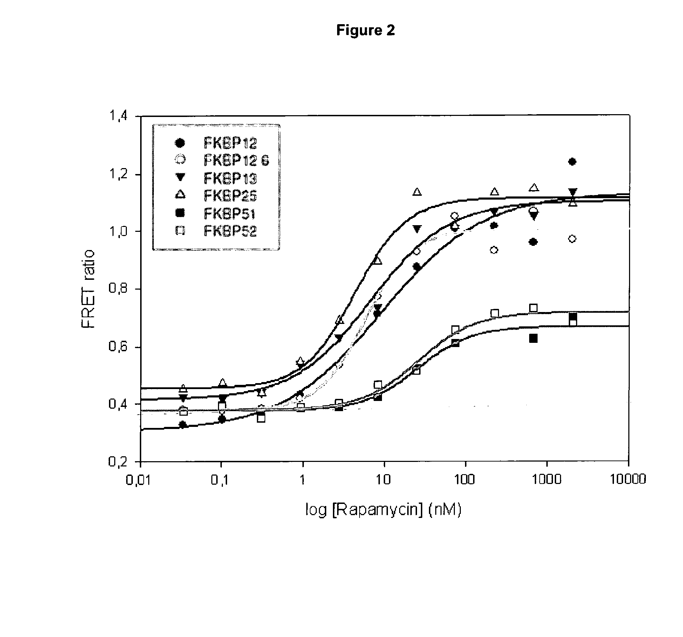

All tested eGFP-FKBPs (FKBP12, FKBP12.6, FKBP13, FKBP25, FKBP51, FKBP52) displayed tight binding to GST-FRB in a clearly rapamycin-dependent manner (Tab. 1, Fig. 2). EC₅₀ values ranged from 3.8 nM for FKBP12 to 25.5 nM for FKBP52. For the larger proteins FKBP51 and FKBP52, binding was slightly weaker and FRET ratio was lower compared to the smaller FKBPs, possibly due to a larger distance or altered orientation of the eGFP tail.

**Table 1: Different FKBPs bind to FRB and inhibit mTOR in an in vitro assay**

| Protein | EC₅₀ (nM) (A) | IC₅₀ (nM) (B) |
|---|---|---|
| FKBP12 | 3.8 ± 0.6 | 0.69 ± 0.17 |
| FKBP12.6 | 5.6 ± 0.9 | 4.79 ± 1.76 |
| FKBP13 | 6.7 ± 1.6 | 2.48 ± 0.82 |
| FKBP25 | 4.3 ± 0.9 | 2.61 ± 0.57 |
| FKBP51 | 25 ± 6.7 | 1.95 ± 0.36 |
| FKBP52 | 25.5 ± 5.8 | 1.68 ± 0.51 |
| FKBP51 FK1 | 10.5 ± 1.8 | |
| FKBP52 FK1 | 26.2 ± 5.6 | |

| | | |
|---|---|---|
| (A) EC₅₀ values of trimeric GST-FRB-rapamycin-eGFP-FKBP complex induction by increasing rapamycin concentrations, determined by a FRET assay (Fig. 2). (B) IC₅₀ values of inhibition of truncated mTOR kinase activity by different FKBP-rapamycin complexes (Fig. 1). | | |

### Example 3: Study on the action of alternative FKBP-rapamycin complexes in eukaryotic cells

To study the action of alternative FKBP-rapamycin complexes in eukaryotic cells a yeast model system lacking the FKBP12 homolog Fpr1, the only cytoplasmatic yeast FKBP, was used (strain BY4742; accession number Y12941; genotype: his3D1; leu2D0; lys2D0; ura3D0;YNL135c::kanMX4). This strain is insensitive to the anti-proliferative effect of rapamycin. Yeast cells were grown on agar plates or in liquid medium containing 0.5% ammonium sulfate, 0.17% yeast nitrogen base and 2% glucose, supplemented with histidine (20 mg/l), lysine (50 mg/l), leucine (60 mg/l) and uracil (20 mg/l). Yeast expression plasmids (pYX223; Novagen/Merck, Darmstadt) containing cDNA of human FKBPs were introduced using standard transformation techniques. After selection of positive clones by histidine auxotrophy, FKBP protein expression was induced by spreading cells on agar containing galactose. Growth of yeast cells on medium containing 5 µM rapamycin was compared to cells growing on normal medium. Thus, their ability to compensate for the missing endogenous FKBP could be determined.

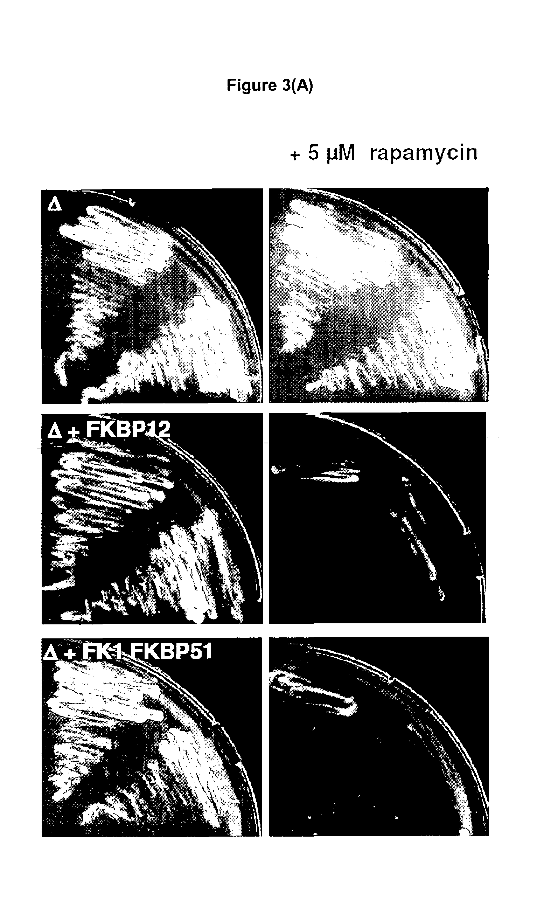

In absence of rapamycin, overexpression of the human FKBPs did not affect yeast growth. However, overexpression of FKBP51 FK1 (rapamycin binding domain of FKBP51) reduced cell growth in the presence of rapamycin to the same extent as observed for FKBP12 (Fig. 3A).

Next mammalian cells were analyzed for a rapamycin-mediated interaction of mTOR and FKBPs. Co-Immunoprecipitation experiments were performed. In detail, the mammalian expression vector pcDNA-3, containing a FLAG-tagged mTOR cDNA sequence, was introduced into HEK-293 cells by standard Lipofectamine transfection. After 48 h, cells were washed twice with TBS and lysed in lysis buffer (40 mM Hepes, pH 7.5; 120 mM NaCl; 1 mM EDTA; 10 mM pyrophosphate; 10 mM glycerophosphate; 0.5 mM orthovanadate; 50 mM NaF; 0.3% (w/v) CHAPS / 1% (v/v) Triton-100; 1% (v/v) protease inhibitor mix) by shaking at 1000 rpm at 4°C for 20 minutes. After 20 minutes of centrifugation at 14.000 rpm and 4°C, the supernatant was collected, and total protein was determined by Bradford Assay. Cell lysates were combined with FLAG affinity resin and incubated under mild agitation for 2 h at 4°C. Beads were pelleted and washed 4 times with lysis buffer. Finally, beads were directly boiled after addition of an equal amount of SDS sample buffer for 5 minutes. Extracts were subjected to SDS-PAGE and immunoblotting.

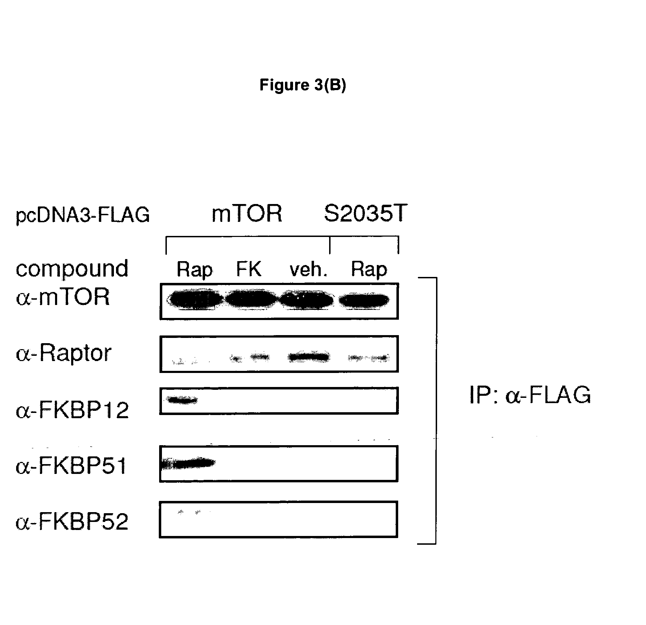

In co-immunoprecipitation experiments with overexpressed mTOR, endogenous FKBP12, FKBP51 and FKBP52 were clearly co-precipitated in the presence of rapamycin (Fig. 3B). No interaction was observed in the absence of rapamycin or in the presence of the related FKBP ligand FK (Tacrolimus). Furthermore, rapamycin was unable to induce an interaction of any of the FKBPs with the mTOR mutant S2035T where the rapamycin-binding site is compromised.

## Claims

1. FKBP subtype-specific rapamycin analogue selective for a single FKBP, wherein the FKBP subtype-specific rapamycin analogue forms a complex with a single FKBP selected from the subtypes FKBP12, FKBP12.6, FKBP13, FKBP19, FKBP22, FKBP23, FKBP25, FKBP36 FKBP38, FKBP51, FKBP52, FKBP60, FKBP65 and FKBP133, wherein the complex binds to the mammalian target of rapamycin for use in treatment or prophylaxis of a disease selected from transplantation/allograft rejection, mTOR-dependent cancers, restenosis, diabetes, insulin resistance, obesity, metabolic syndrome, autoimmune diseases, tuberous sclerosis, depression, cognition and neurological disorder.

2. FKBP subtype-specific rapamycin analogue according to claim 1, wherein the FKBP subtype-specific rapamycin analogue is selected from the group consisting of Biolimus A9, Zotarolimus, Everolimus, Myolimus, Novolimus, Pimecrolimus, Tacrolimus, Ridaforolimus and Temsirolimus.

3. FKBP subtype-specific rapamycin analogue according to claim 1 or 2, wherein the autoimmune disease is systemic lupus erythematosus, Sjogren's syndrome and/or rheumatoid arthritis.

4. FKBP subtype-specific rapamycin analogue according to claim 1 or 2, wherein the neurodegeneration is Parkinson's disease, Alzheimer's disease, Huntington's disease and/or amyotrophic lateral sclerosis.

5. FKBP subtype-specific rapamycin analogue according to any one of the claims 1 - 4, wherein the FKBP subtype-specific rapamycin analogue forms a complex with a single FKBP, wherein the FKBP is FKBP51.

6. FKBP subtype-specific rapamycin analogue according to any of the claims 1 - 4, wherein the FKBP subtype-specific rapamycin analogue is selective for two FKBPs and the FKBP subtype-specific rapamycin analogue forms a complex with one of the two FKBPs selected from the subtypes FKBP12, FKBP12.6, FKBP13, FKBP19, FKBP22, FKBP23, FKBP25, FKBP36 FKBP38, FKBP51, FKBP52, FKBP60, FKBP65 and FKBP133.

7. FKBP subtype-specific rapamycin analogue according to any of the claims 1 - 4, wherein the FKBP subtype-specific rapamycin analogue is selective for three FKBPs and the FKBP subtype-specific rapamycin analogue forms a complex with one of the three FKBPs selected from the subtypes FKBP12, FKBP12.6, FKBP13, FKBP19, FKBP22, FKBP23, FKBP25, FKBP36 FKBP38, FKBP51, FKBP52, FKBP60, FKBP65 and FKBP133.

8. FKBP subtype-specific rapamycin analogue according to any of the claims 1 - 4, wherein the FKBP subtype-specific rapamycin analogue is selective for four FKBPs and the FKBP subtype-specific rapamycin analogue forms a complex with one of the four FKBPs selected from the subtypes FKBP12, FKBP12.6, FKBP13, FKBP19, FKBP22, FKBP23, FKBP25, FKBP36 FKBP38, FKBP51, FKBP52, FKBP60, FKBP65 and FKBP133.

9. FKBP subtype-specific rapamycin analogue according to claim 6, wherein the FKBP subtype-specific rapamycin analogue is selective for FKBP12 and FKBP52 and/or FKBP12 and KFBP51 and/or FKBP51 and FKBP52.

10. FKBP subtype-specific rapamycin analogue according to claim 7, wherein the FKBP subtype-specific rapamycin analogue is selective for FKBP12, FKBP12.6 and KFBP52 and/or FKBP12, FKBP51 and FKBP52 and/or FKBP12, FKBP38 and FKBP51.

11. FKBP subtype-specific rapamycin analogue according to claim 8, wherein the FKBP subtype-specific rapamycin analogue is selective for FKBP12, FKBP12.6, FKBP51 and FKBP52 and/or FKBP12, FKBP38, FKBP51 and FKBP52.
